# EUROPEAN PATENT APPLICATION

(11) **EP 2 409 710 A1**
(43) Date of publication of application: **25.01.2012**
(21) Application number: 10167744.1
(22) Date of filing: 29.06.2010
(51) Int. Cl.: A61K 38/39, A61K 38/43, A61K 9/50, A61P 19/10

(54) **Injectable material and material to be used as drug or food supplement for prophylaxis or treatment of osteoporosis**

(71) Applicant: NanotecMARIN GmbH, 55099 Mainz (DE)
(72) Inventor: Müller, Werner E.G., Prof. Dr., 65203 Wiesbaden (DE); Wang, Xiaohong, Prof. Dr., 65189 Wiesbaden (DE); Wiens, Matthias, Dr., 55270 Essenheim (DE)
(74) Representative: Krauss, Jan

(57) **Abstract**

The invention concerns the application of a novel biosilica-based material for prophylaxis and treatment of osteoporosis, which acts by increasing the osteoprotegerin : RANKL ratio and can be administered by percutaneous injection or used as a drug or food supplement.

SEQUENCE LISTING

<110>  NanotecMARIN GmbH

<120>  INJECTABLE MATERIAL AND MATERIAL TO BE USED AS DRUG OR FOOD
       SUPPLEMENT FOR PROPHYLAXIS OR TREATMENT OF OSTEOPOROSIS

<130>  FB23351

<160>  4

<170>  PatentIn version 3.5

<210>  1
<211>  330
<212>  PRT
<213>  Suberites domuncula

<400>  1

Met Leu Val Thr Val Val Val Leu Gly Leu Leu Gly Phe Ala Ser Ala
1               5                   10                  15

Ala Gln Pro Lys Phe Glu Phe Val Glu Glu Trp Gln Leu Trp Lys Ser
            20                  25                  30

Thr His Ser Lys Met Tyr Glu Ser Gln Leu Met Glu Leu Glu Arg His
        35                  40                  45

Leu Thr Trp Leu Ser Asn Lys Lys Tyr Ile Glu Gln His Asn Val Asn
    50                  55                  60

Ser His Ile Phe Gly Phe Thr Leu Ala Met Asn Gln Phe Gly Asp Leu
65                  70                  75                  80

Ser Glu Leu Glu Tyr Ala Asn Tyr Leu Gly Gln Tyr Arg Ile Glu Asp
                85                  90                  95

Lys Lys Ser Gly Asn Tyr Ser Lys Thr Phe Gln Arg Asp Pro Leu Gln
            100                 105                 110

Asp Tyr Pro Glu Ala Val Asp Trp Arg Thr Lys Gly Ala Val Thr Ala
        115                 120                 125

Val Lys Asp Gln Gly Asp Cys Gly Ala Ser Tyr Ala Phe Ser Ala Met
    130                 135                 140

Gly Ala Leu Glu Gly Ala Asn Ala Leu Ala Lys Gly Asn Ala Val Ser
145                 150                 155                 160

Leu Ser Glu Gln Asn Ile Ile Asp Cys Ser Ile Pro Tyr Gly Asn His
                165                 170                 175

Gly Cys His Gly Gly Asn Met Tyr Asp Ala Phe Leu Tyr Val Ile Ala
            180                 185                 190

Asn Glu Gly Val Asp Gln Asp Ser Ala Tyr Pro Phe Val Gly Lys Gln
        195                 200                 205

Ser Ser Cys Asn Tyr Asn Ser Lys Tyr Lys Gly Thr Ser Met Ser Gly
    210                 215                 220

Met Val Ser Ile Lys Ser Gly Ser Glu Ser Asp Leu Gln Ala Ala Val
225                 230                 235                 240

Ser Asn Val Gly Pro Val Ser Val Ala Ile Asp Gly Ala Asn Ser Ala
                245                 250                 255

Phe Arg Phe Tyr Tyr Ser Gly Val Tyr Asp Ser Ser Arg Cys Ser Ser
            260                 265                 270

Ser Ser Leu Asn His Ala Met Val Val Thr Gly Tyr Gly Ser Tyr Asn
        275                 280                 285

Gly Lys Lys Tyr Trp Leu Ala Lys Asn Ser Trp Gly Thr Asn Trp Gly
    290                 295                 300

Asn Ser Gly Tyr Val Met Met Ala Arg Asn Lys Tyr Asn Gln Cys Gly
305                 310                 315                 320

Ile Ala Thr Asp Ala Ser Tyr Pro Thr Leu
                325                 330

<210>  2
<211>  1214
<212>  DNA
<213>  Suberites domuncula

<400>  2
aggatagaaa gtctacaatc tgtaaggaca atgcttgtca cagtggtagt actgggtcta      60

ctggggtttg cttctgcagc ccagcccaag tttgaatttg tagaagaatg gcagctgtgg     120

aagtccactc actctaagat gtacgagtca cagttaatgg aactcgaaag acatctgacg     180

tggctctcca ataagaaata tatcgagcaa cacaatgtca actcacacat tttcggtttt     240

actctggcaa tgaaccagtt tggagatctg agtgaattgg agtatgctaa ctatcttggc     300

cagtatcgca ttgaggataa aaaatctggc aactactcaa agacttttca gcgtgatcct     360

ctacaggact accctgaagc tgtagactgg agaaccaaag gagctgtcac ggctgtcaag     420

gaccagggag actgtggtgc tagctatgct ttcagtgcta tgggtgcttt ggagggtgct     480

aatgctttag ccaagggaaa tgcagtatct ctcagtgaac agaacatcat tgattgctcg     540

attccttacg gtaaccacgg ttgtcatgga ggcaatatgt atgatgcttt tttgtatgtc     600

atcgctaacg agggggtcga tcaggacagt gcatatccat ttgtaggaaa gcaatccagc     660

tgcaactata atagtaaata caaaggtaca tcaatgtcgg ggatggtgtc aatcaaaagt     720

ggtagtgagt ctgacttaca agcagctgtt tcaaacgttg gccctgtatc tgttgctatt     780

gatggtgcta acagtgcctt caggttttac tacagtggtg tctatgactc atcacgatgc     840

tctagtagta gtcttaacca cgcaatggta gtcactggat acggatcata caatgggaaa     900

aaatactggc tggccaagaa tagctgggga actaactggg gtaacagtgg ctatgtgatg 960

atggctcgca acaagtacaa ccagtgtgga attgctaccg atgcatctta tcccacccta 1020

taaacttata tatatatagt cttagaaaca ttatcctttt ctttaccctt gtctctatag 1080

gccatagagt gattgtaggc tgtttgcatt tgatgactgt atatacccta tcattttttg 1140

tgattctatc tgattaaaaa tcccataccc gaccaaacca tcaatttatc aaatcatgaa 1200

aaaaaaaaaa aaaa 1214

<210> 3
<211> 386
<212> PRT
<213> Suberites domuncula

<400> 3

Met Ser Ser Glu Glu Thr Pro Val Glu Gln Pro Thr Ala Glu Glu Thr
1               5                   10                  15

Pro Ala Ser Thr Glu Glu Thr Pro Ala Glu Thr Pro Ala Thr Thr Glu
            20                  25                  30

Thr Thr Glu Pro Ala Ala Asp Asp Gln Pro Ala Val Thr Glu Val Thr
        35                  40                  45

Glu Val Thr Glu Thr Pro Ala Glu Pro Thr Pro Glu Ala Thr Thr Glu
    50                  55                  60

Glu Pro Lys Ala Glu Glu Lys Lys Glu Glu Val Lys Gln Lys Pro Lys
65                  70                  75                  80

Lys Glu Pro Lys Pro Ala Pro Asp Tyr Arg Thr Thr Lys Ser Gly Trp
                85                  90                  95

Val Gln Lys Gln Gly Leu Leu Phe Lys Lys Trp Gln Arg Gln Phe Met
            100                 105                 110

Thr Leu Asp Glu Glu Asp Ser Val Leu Arg Trp Trp Lys Ser Glu Gln
        115                 120                 125

Arg Thr Gly Ser Asp Gly Ala Leu Tyr Met Lys Phe Cys Val Gly Val
    130                 135                 140

Ser Asp Pro Lys Ala Glu Thr Thr Thr Thr Trp Pro Asp Asp Thr Ala
145                 150                 155                 160

Asp Arg Cys Phe Val Val Cys Ser Pro Tyr Arg Ala Phe Phe Leu Val
                165                 170                 175

Ala Glu Ser Gln Glu Asp Lys Asn Asp Trp Ile Gln Lys Leu Ser Ala
            180                 185                 190

Ala Lys Asp Lys His Asn Thr Glu Gly Arg Lys Leu Ile Thr Thr Ala
        195                 200                 205

Leu Ile Ser Val Glu Gly Ile Lys Ala Ser Phe Asp Asn Gly Ala Ala
    210                 215                 220

Asp Leu Asp Lys Thr Val Lys Lys Glu Gly Ile Val Glu Glu Lys Lys
225                 230                 235                 240

Thr Glu Glu Leu Leu Gln Leu Asp Lys Thr Val Lys Lys Glu Gly Ile
                245                 250                 255

Val Glu Glu Lys Lys Thr Glu Glu Ala Ala Pro Thr Glu Ser Glu Thr
            260                 265                 270

Ala Pro Ala Glu Ala Glu Gly Ala Ala Ala Val Thr Ile Glu Val Glu
        275                 280                 285

Val Glu Thr Pro Ala Glu Glu Pro Pro Lys Glu Glu Thr Pro Ala Glu
    290                 295                 300

Glu Pro Pro Lys Glu Glu Thr Pro Ala Glu Val Pro Pro Lys Glu Glu
305                 310                 315                 320

Thr Pro Ala Glu Glu Pro Pro Lys Glu Glu Thr Pro Ala Glu Glu Pro
                325                 330                 335

Pro Lys Glu Glu Thr Pro Ala Glu Glu Pro Pro Lys Glu Glu Thr Pro
            340                 345                 350

Ala Glu Asp Pro Pro Lys Glu Glu Glu Thr Pro Ala Glu Glu Pro Pro
        355                 360                 365

Lys Asp Glu Ala Pro Ala Ala Ala Glu Gly Gly Glu Glu Thr Lys Ala
    370                 375                 380

Glu Asp
385

<210>  4
<211>  1611
<212>  DNA
<213>  Suberites domuncula

<400>  4
gggggatagg ggaaaattca tttctgcaga gttcagtgca gtaaagaaaa gaaggctaag      60

atatccagca tctcgaaaac tatctatctc aagcacttac accatgtcat cagaagagac     120

cccagtagaa cagcccacag ctgaggagac gccagcctct acagaggaga caccagcaga     180

gaccccagct accactgaaa caacagagcc agctgctgat gaccagcctg ctgtcacaga     240

ggtcacagag gtcactgaga ctccagccga gcctactcca gaagccacca cagaggagcc     300

caaggctgaa gagaagaaag aagaagtcaa acagaagccc aagaaggaac ccaagcctgc     360

cccagactac agaacaacaa agagtggctg ggttcagaaa caaggactgc tcttcaagaa     420

gtggcagcga cagttcatga ccctggatga ggaggatagt gtactaagat ggtggaagtc 480
tgaacaaagg actggctctg atggcgctct ctatatgaag ttctgtgttg gtgtgtctga 540
ccccaaggct gaaacaacaa ccacttggcc tgatgacaca gctgatcgat gctttgttgt 600
gtgctctcct tacagagcat tcttcctagt agcagagagt caagaggata agaatgactg 660
gatccagaaa ctttctgctg ccaaagataa gcacaacact gaaggccgta agctgatcac 720
tactgccctg atctctgtgg aaggcatcaa ggcaagtttt gataatggag ctgctgacct 780
ggacaagact gtcaagaaag agggcattgt tgaggagaag aagacagagg agctgctcca 840
actggacaag actgtcaaga aagagggcat tgttgaggag aagaagacag aggaggctgc 900
tccaactgag agtgaaacag ctcctgcaga ggcagaaggt gctgctgcag ttactataga 960
agttgaggtt gaaacaccag ctgaagagcc ccctaaagaa gaaactccag cagaggagcc 1020
acccaaagag gagacacccg cagaggttcc ccccaaagag gagacacccg cagaggaacc 1080
ccccaaagag gaaacacctg ctgaggagcc ccccaaagag gagacacccg cagaagaacc 1140
ccccaaagag gagacgcctg cagaggatcc ccctaaagaa gaagaaacac cagctgagga 1200
accaccaaag gatgaagcac cagcagcagc tgaaggaggt gaggagacaa aagcagaaga 1260
ctaaataaac tgtagcacac gataggatta ttattgtgga caaatactgt ttaaaaattg 1320
acgctatcag atcaaaagac ttacacgcac ataattatta ccttccacta cacttattag 1380
tttgttacat gtacaaatag atgcacattt ttgtattgta cactttttat acaaaggttt 1440
gtgttgttat tcctgtatgt gtatgatatg gatttttgtt tattctatag tacatggtcc 1500
ccttaattct gatttagaaa cttttctgta cattgagcag ttttactcca cttttagtgt 1560
gctgtgtgta atattatcaa attttgaata ataaatgttt tttgctgcaa a 1611

## Description

The invention concerns the application of a novel biosilica-based material for prophylaxis and treatment of osteoporosis, which acts by increasing the osteoprotegerin : RANKL ratio and can be administered by percutaneous injection or used as a drug or food supplement.

### Background of Invention

Osteoporosis is the most common metabolic bone disorder. Over 200 million people suffer from osteoporosis worldwide. The costs associated with osteoporosis are enormous. In Europe, the (estimated) costs of fractures caused by osteoporosis amount to about 30 billion Euros per year. These costs are increasing because of the increasing proportion of elderly people in the population of many countries.

Osteoporosis is a systemic skeletal disorder characterized by a low bone mass and by microarchitectural deterioration of bone tissue, with a subsequent increase in bone fragility and susceptibility to fracture (WHO definition). It is characterized by reduced bone strength and bone mineral density due to an imbalance between bone resorption and formation. Osteoporosis is most common in women after menopause but may also develop in men (reviewed in: Raisz LG. Pathogenesis of osteoporosis: concepts, conflicts, and prospects. J Clin Invest 115:3318-3325, 2005). In particular hormonal disorders contribute to bone-loss and reduced activity/number of osteoblasts. In addition, nutritional factors have been identified as etiological cause for osteoporosis (reviewed in: Jugdaohsingh R. Silicon and bone health. J Nutr Health Aging 11:99-110, 2007).

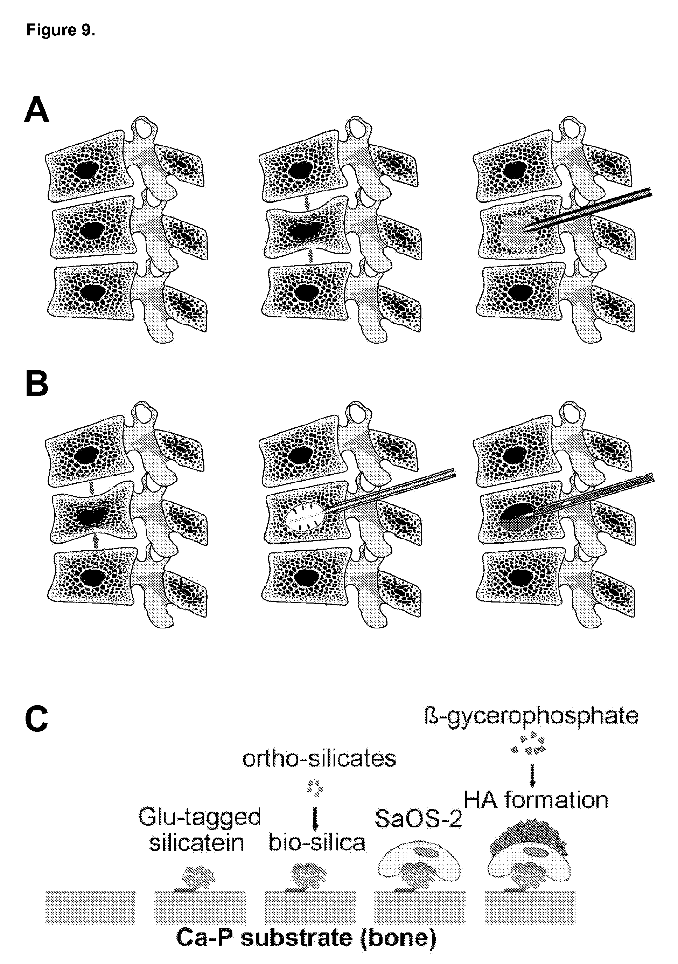

The progressive deterioration of the microarchitecture of bone tissue in osteoporosis particularly concerns cancellous (trabecular) bone, resulting in shrinkage of cortical width, rarefaction of the trabecular network and increased occurrence of disconnected trabeculae due to increased osteoclastic resorption. The progressive loss of both cortical bone and trabecular bone in particular results in an increased risk of hip fractures (fractures of the proximal femur) and vertebral fractures (compression fractures: depression of the upper and lower cortices of the vertebral bodies; see also Figure 9A,B). Hip fractures account for about 65% of the total medical costs of osteoporotic fractures (more than 600,000 patients annually in Europe and more than 300,000 patients in the USA). Vertebral fractures are found in 5-10% of women at an age of 55 years and 30-40% of 80-years old women.

Current treatments of osteoporosis include:
- Calcium and vitamin D (supplements)
- Bisphosphonates: alendronate, risedronate, ibandronate, and zoledronate
- Selective oestrogen-receptor modulators: raloxifene
- Parathyroid hormone (PTH): teriparatide (PTH fragment)
- Strontium ranelate

Bisphosphonates are synthetic analogous of pyrophosphate in which the oxygen atom of the P-O-P bond has been replaced by carbon. The resulting P-C-P group makes the bisphosphonates resistant to enzymatic hydrolysis. Various compounds are available, which are obtained by different substitution of the hydrogen atoms on the carbon atom of the P-C-P group. Therapy with bisphosphonates has recently been associated with the occurrence of osteonecrosis of the jaw. Bisphosphonates may inhibit the degradation of polyphosphate which can be given in combination with the biosilica material described by the inventors (Leyhausen G, Lorenz B, Zhu H, Geurtsen W, Bohnensack R, Müller WE, Schröder HC. Inorganic polyphosphate in human osteoblast-like cells. J Bone Miner Res 13:803-812, 1998).

### The RANK/RANKL/OPG system

Osteoprotegerin (OPG) and receptor activator for NF-KB ligand (RANKL) are one of the most crucial signaling systems that mediate and modulate bone resorption (reviewed in: Lane NE, Yao W. Developments in the scientific understanding of osteoporosis. Arthritis Res Ther 11:228, 2009). The crosstalk between osteoblasts (bone-producing cells), mediated by cellular and soluble factors, controls the differentiation of pre-osteoclasts to mature osteoclasts (bone-resorbing cells). On the cellular level the following three members of the tumor necrosis factor (TNF) and TNF receptor superfamily are involved (reviewed in: Khosla S. Minireview: The OPG/RANKL/RANK System. Endocrinology 142:5050-5055, 2001):
*(i)* RANK, the receptor activator of NF-KB, that is expressed on hematopoietic cells and controls osteoclastogenesis (osteoclast maturation),
*(ii)* OPG that is secreted by osteoblasts and blocks osteoclastogenesis and thus inhibits bone resorption, and
*(iii)* RANKL (ligand of receptor activator for NF-KB, RANK), a protein produced by osteoblasts, which activates osteoclasts through interaction with RANK on osteoclast precursor cells.

The RANKL/RANK interaction plays a key role in the differentiation and maintenance of osteoclast activity, and thus is fundamentally involved in the manifestation of osteoporosis. The effect of RANKL is regulated by OPG. Therefore, the relative concentrations of OPG and RANKL are crucial for the development of this disease.

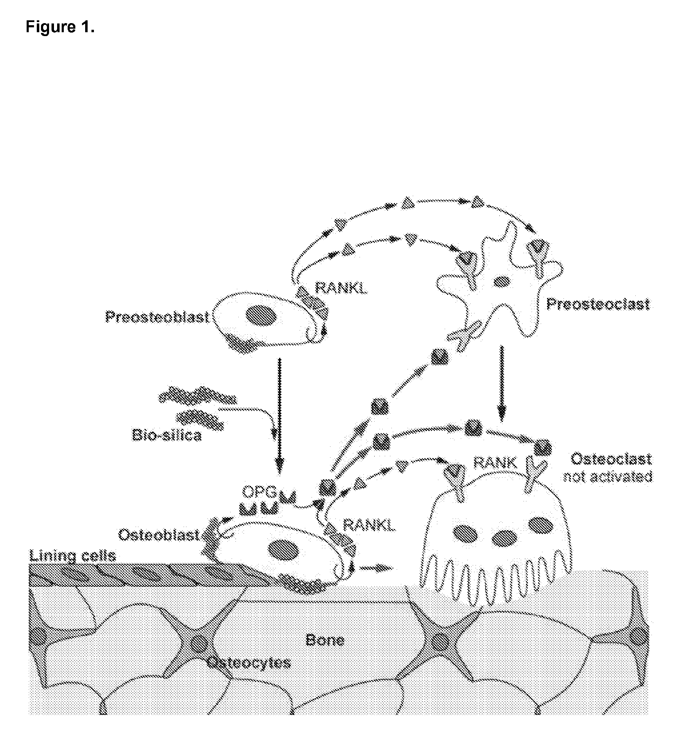

A schematic presentation of the RANK/RANKL/OPG system and of the effect of the biosilica material described by the inventors on this system is depicted in Figure 1.

Differentiation of osteoblasts also requires the expression of bone morphogenetic protein 2 (BMP2; Tanaka H, Nagai E, Murata H, Tsubone T, Shirakura Y, Sugiyama T, Taguchi T, Kawai S. Involvement of bone morphogenic protein-2 (BMP-2) in the pathological ossification process of the spinal ligament. Rheumatology 40:1163-1168, 2001). BMP2 is a member of the transforming growth factor-ß (TGFß) superfamily.

The tartrate-resistant acid phosphatase (TRAP) is expressed by osteoclasts; lower levels can be found in SaOS-2 cells. TRAP is a modulator of bone resorption and its increased expression is associated with osteoporosis and other bone diseases (Oddie GW, Schenk G, Angel NZ, Walsh N, Guddat LW, de Jersey J, Cassady Al, Hamilton SE, Hume DA. Structure, function, and regulation of tartrate-resistant acid phosphatase. Bone 27:575-584, 2000).

### Silicon - Biosilica

In its oxidized form, silicon (Si) comprises almost exclusively a tetrahedral coordination, with 4 oxygen (O) atoms that surround the central Si atom, and occurs as SiO₂ in the thermodynamically most stable crystalline form (Perry CC. Silicification: the processes by which organisms capture and mineralize silica. Rev Mineral Geochem 54:291-327, 2003). In the living world SiO₂ occurs as amorphous biosilica, in particular in plants, algae, and sponges (reviewed in: Müller WEG. Silicon Biomineralization: Biology-Biochemistry-Molecular Biology-Biotechnology. Springer: Berlin Heidelberg, 2003). The monomeric form of silica, orthosilicate, is water-soluble and has the pKₐ of a weak acid, 9.6. At neutral pH, it has the tendency to polymerize to poly(silicate), particularly at concentrations above 2-3 mM (Iler RK. Solubility, polymerisation, colloid and surface properties, and biochemistry. John Wiley & Sons, New York, 1979). Silicic acid is taken up by cells of plants, algae, and sponges (Schröder HC, Perovic-Ottstadt S, Rothenberger M, Wiens M, Schwertner H, Batel R, Korzhev M, Müller IM, Müller WEG. Silica transport in the demosponge Suberites domuncula: Fluorescence emission analysis using the PDMPO probe and cloning of a potential transporter. Biochem J 381:665-673, 2004) and provides the basis for intricate bio-siliceous exoskeletons (reviewed in: Schröder HC, Wang XH, Tremel W, Ushijima H, Müller WEG. Biofabrication of biosilica-glass by living organisms. Nat Prod Rep 25:455-474, 2008).

In the human body, the concentration of Si/Si(OH)₄ is comparably low (1-2 g totally) (reviewed in: Jugdaohsingh R. Silicon and bone health. J Nutr Health Aging 11:99-110, 2007). Si is taken up via gastrointestinal absorption. After circulation through the blood, Si/Si(OH)₄ is eliminated via the kidney into the urine. Highest silicon concentrations are found in connective tissue, bone, and blood vessels. Si/Si(OH)₄ is present in particular in active calcification sites during bone formation. Based on epidemiological studies, it has been proposed that the level of silicon is associated with higher bone mineral density (BMD) and strength (reviewed in: Jugdaohsingh R. Silicon and bone health. J Nutr Health Aging 11:99-110, 2007).

Silicic acid [Si(OH)₄] has been shown to promote proliferation of osteoblasts, extracellular matrix differentiation, and gene expression, and to increase alkaline phosphatase activity (Reffitt DM, Ogston N, Jugdaohsingh R, Cheung HF, Evans BA, Thompson RP, Powell JJ, Hampson GN. Orthosilicic acid stimulates collagen type I synthesis and osteoblastic differentiation in human osteoblast-like cells in vitro. Bone 32:127-135, 2003; Arumugam MQ, Ireland DC, Brooks RA, Rushton N, Bonfield W. The effect of orthosilicic acid on collagen type I, alkaline phosphatase and osteocalcin mRNA expression in human bone-derived osteoblasts in vitro. Key Eng Mater 32:309-311, 2006). Consequently, application of Si-containing implant materials and ceramics has been studied (reviewed in: Chen QZ, Thompson ID, Boccaccini AR. 45S5 Bioglass®-derived glass-ceramic scaffolds for bone tissue engineering. Biomaterials 27:2414-2425, 2006; López-Alvarez M, Solla EL, González P, Serra J, León B, Marques AP, Reis RL. Silicon-hydroxyapatite bioactive coatings (Si-HA) from diatomaceous earth and silica. J Mater Sci Mater Med 20:1131-1136, 2009; Zou S, Ireland D, Brooks RA, Rushton N, Best S. The effects of silicate ions on human osteoblast adhesion, proliferation, and differentiation. J Biomed Mater Res B Appl Biomater 90:123-130, 2009).

Biosilica (amorphous, hydrated silicon oxide) is the inorganic component of the skeleton of siliceous sponges. The discovery of silicatein, an enzyme from marine and freshwater sponges, has provided a fundamental molecular tool to harness the biogenous synthesis of biosilica; silicatein is the first enzyme at all that is able to catalyze the formation of an inorganic polymer (here: silica - biosilica) (Shimizu K, Cha J, Stucky GD, Morse DE. Silicatein alpha: cathepsin L-like protein in sponge biosilica. Proc Natl Acad Sci USA 95:6234-6238, 1998; Cha et al. 1999; Krasko A, Batel R, Schröder HC, Müller IM, Müller WEG. Expression of silicatein and collagen genes in the marine sponge Suberites domuncula is controlled by silicate and myotrophin. Eur J Biochem 267:4878-4887, 2000). Silicatein is able to form biosilica following an enzymatic process; the catalytic constants have been determined (Müller WEG, Schloßmacher U, Wang XH, Boreiko A, Brandt D, Wolf SE, Tremel W, Schröder HC. Silicatein in siliceous spicules and silicasomes of demosponges comprises dual enzymatic activities (silica-polymerase and silica-esterase). FEBS J 275:362-370, 2008).

Several isoforms of silicatein have been identified. Silicatein-α and the application of this enzyme in various areas of technology and biomedicine have been patented by the inventors (German Patent No. DE10037270**,** European Patent No. EP1320624**,** United States Patent No. US 7,169,589B2**,** Chinese Patent No. ZL01813484.X**,** New Zealand Patent **No.**523474**,** Australia Patent No. 2001289713**.** Silicatein-mediated synthesis of amorphous silicates and siloxanes and their uses. Inventors: Müller WEG, Lorenz A, Krasko A, Schröder HC; national phases: NO20030407; Japan No. 2002-516336; Canada No. 2,414,602). Review articles: Müller WEG, Wang X, Belikov SI, Tremel W, Schloßmacher U, Natoli A, Brandt D, Boreiko A, Tahir MN, Müller IM, Schröder HC Formation of siliceous spicules in demosponges: example Suberites domuncula. In: Handbook of Biomineralization. Vol 1: Biological Aspects and Structure Formation (ed by E Bäuerlein). Wiley-VCH, Weinheim, pp 59-82, 2007; Schröder HC, Brandt D, Schloßmacher U, Wang X, Tahir MN, Tremel W, Belikov Sl, Müller WEG. Enzymatic production of biosilica glass using enzymes from sponges: basic aspects and application in nanobiotechnology (material sciences and medicine). Naturwissenschaften 94:339-359, 2007; Schröder HC, Wang X, Tremel W, Ushijima H, Müller WEG. Biofabrication of biosilica-glass by living organisms. Nat Prod Rep 25:455-474, 2008.

Besides silicatein-α (patents see above), further silicateins were cloned by the inventors, including silicatein-β (patent application: DE10352433.9**.** Enzym- und Template-gesteuerte Synthese von Silica aus nicht-organischen Siliciumverbindungen sowie Aminosilanen und Silazanen und Verwendung. Inventors: Schwertner H, Müller WEG, Schröder HC), and four silicatein isoforms from a freshwater sponge (silicatein-a1-4; DE102006001759.5. Kontrollierte Herstellung von Silber- und Gold-Nanopartikeln und Nanokristallen definierter Größe und Form durch chirale Induktion mittels Silicatein. Inventors: Tremel W, Tahir MN, Müller WEG, Schröder HC). In addition, silicatein from the marine sponge *Tethya lyncurium* has been patented **(**PCT/US99/30601**.** Methods, compositions, and biomimetic catalysts, such as silicateins and block copolypeptides, used to catalyze and spatially direct the polycondensation of silicon alkoxides, metal alkoxides, and their organic conjugates to make silica, polysiloxanes, polymetallo-oxanes, and mixed poly(silicon/metallo)oxane materials under environmentally benign conditions. Inventors: Morse DE, Stucky GD, Deming, TD, Cha J, Shimizu K, Zhou Y).

Another protein involved in silica metabolism is the silicatein interactor protein, silintaphin-1 (European Patent Application EP09005849.6**.** Use of silintaphin for the structure-directed fabrication of (nano)composite materials in medicine and (nano)technology. Inventors: Wiens M, Müller WEG, Schröder HC, Wang X). Silintaphin-1 is a silicatein-binding protein which has a structure-directing activity: it directs the assembly of silica nanoparticles to 3-dimensional silica structures. Silintaphin-1 forms the "core" of the natural silicatein filaments ("axial filaments") of the sponge spicules. The silintaphin-1 sequence shows no homologies to any other known protein. Silintaphin-1 is also able to assemble, besides biosilica, nanoparticles of other metal oxides (Wiens M, Bausen M, Natalio F, Link T, Schlossmacher U, Müller WEG. The role of the silicatein-alpha interactor silintaphin-1 in biomimetic biomineralization. Biomaterials 30:1648-1656, 2009).

The state of the art in the enzymatic and structure-directed synthesis of biosilica has been described in: Schröder HC, Wang XH, Tremel W, Ushijima H, Müller WEG. Biofabrication of biosilica-glass by living organisms. Nat Prod Rep 25:455-474, 2008; Müller WEG, Wang XH, Cui FZ, Jochum KP, Tremel W, Bill J, Schröder HC, Natalio F, Schloßmacher U, Wiens M. Sponge spicules as blueprints for the biofabrication of inorganic-organic composites and biomaterials. Appl Microbiol Biotechnol 83:397-413,2009.

The following publications are relevant with regard to the application of biosilica in tissue engineering / preparation of bone replacement materials / coating of medical implants:
1. Krasko A, Batel R, Schröder HC, Müller IM, Müller WEG. Expression of silicatein and collagen genes in the marine sponge Suberites domuncula is controlled by silicate and myotrophin. Eur J Biochem 267:4878-4887, 2000.
2. Schröder HC, Boreiko A, Krasko A, Reiber A, Schwertner H, Müller WEG. Mineralization of SaOS-2 cells on enzymatically (Silicatein) modified bioactive osteoblast-stimulating surfaces. J Biomed Mater Res B Appl Biomater 75B:387-392, 2005.
3. Müller WEG, Boreiko A, Wang XH, Krasko A, Geurtsen W, Custódio MR, Winkler T, Lukić-Bilela L, Link T, Schröder HC. Morphogenetic activity of silica and biosilica on the expression of genes, controlling biomineralization using SaOS-2 cells. Calcif Tissue lnt 81:382-393, 2007.

The following patent applications on the application of biosilica in biomedicine and dentistry are pending:
1. Patent applications: DE 102004021229.5**;** EP 2005004738**;** US 11579020**;** JP 2007509992**;** CA2565121. Enzymatic method for producing bioactive, osteoblast-stimulating surfaces and use thereof. Inventors: Müller WEG, Schwertner H, Schröder HC.
2. Patent applications: US 60839601**;** EP 2007007363. Biosilica-adhesive protein nano-composite materials: synthesis and application in dentistry. Inventors: Müller WEG, Schröder HC,Geurtsen WK.
3. US Patent application PCT/US2009/005302**.** Compositions, oral care products and methods of making and using the same. Inventors: Miller J, Höfer H, Geurtsen W, Lücker P, Wiens M, Schröder HC, Müller WEG.

In view of the background art as described above, the object of the present invention was to provide improved means for the prophylaxis and/or the treatment of patients suffering from bone disorders, in particular osteoporosis.

### Brief Description of the Invention:

The above object is solved in a first aspect by an injectable material or material to be used as a drug or food supplement for the prophylaxis or treatment of osteoporosis, consisting of an enzyme or protein involved in biosilica (amorphous, hydrated silicon oxide) metabolism, such as silicatein or silintaphin-1 or silicatein-silintaphin-1 fusion proteins or combinations thereof, wherein said protein is provided with an N-terminal or C-terminal bound apatite (calcium phosphate) or silica-binding oligocationic or oligoanionic tag, or a multiple thiol groups carrying tag.

In a next embodiment the oligocationic or oligoanionic tag consists of an oligo(amino acid), which preferably is composed of glutamic acid, aspartic acid or lysine or mixtures of these amino acids, for example of a length of six to ten amino acids.

According to the present invention the multiple thiol groups carrying tag of the inventive injectable material consists of an oligo(cysteine) residue.

In one embodiment of the invention the enzyme or protein involved in biosilica metabolism carries a tag on both its N-terminus and C-terminus, whereby the chemical nature of these two tags (oligocationic or oligoanionic tag, or multiple thiol groups carrying tag) is either different or the same. According to the invention a silicatein polypeptide or a silicatein fusion protein is used, which has been produced using a prokaryotic or eukaryotic expression system, or which has been produced synthetically.

Yet in another embodiment of the invention a silintaphin-1 polypeptide or a silintaphin-1 fusion protein is used, wherein said silintaphin-1 polypeptide or a polypeptide being homologous thereto in the amino acid sequence of the silintaphin-1 domain exhibits a sequence similarity or identity of at least 25% to the sequence shown in SEQ ID Nr. 3, or parts thereof.

Also preferred in the context of the present invention is that a silicatein polypeptide is used, or a polypeptide being homologous thereto, which in the amino acid sequence of the silicatein domain exhibits a sequence similarity or identity of at least 25% to the sequence shown in SEQ ID Nr. 1, or parts thereof.

In a further preferred embodiment of the invention the tagged silicatein molecules or tagged silintaphin-1 molecules are bound via their tag to hydroxyapatite (HA) nanoparticles or HA - collagen nanoparticles.

According to the present invention an injectable material is preferred which has been modified ex vivo by biosilica formation using its silicatein or silicatein fusion protein component and a suitable biosilica precursor, wherein the biosilica precursor preferably consists of water glass, orthosilicic acid, orthosilicates, monoalkoxysilanetriols, dialkoxysilanediols, dialkoxysilanols, trialkoxysilanols, tetraalkoxysilanes, alkyl- or aryl-silanetriols, alkyl- or aryl-silanediols, alkyl- or aryl-monoalkoxysilanediols, alkyl- or aryl-monoalkoxysilanols, alkyl- or aryl-dialkoxysilanols, alkyl- or aryl-trialkoxysilanes, or silicon-catecholate complexes.

Particularly preferred is an injectable material which has been modified ex vivo by binding of nanoparticulate silica particles (nanosilica) or microparticulate silica particles or other metal oxide particles or mixtures thereof to its silintaphin-1 or silintaphin-1 fusion protein component. Preferably the injectable material has been modified ex vivo by binding of iron [iron (II) and/or iron (III)] to the multiple thiol groups carrying tag.

Another embodiment of the invention relates to an injectable material, consisting of the polymeric inorganic components (biosilica, oligo- and polysilicates), separated from the organic component.

An injectable material of the invention is preferred, whereby one or more of the enzyme or protein components (silicatein or silintaphin-1 or silicatein-silintaphin-1 fusion proteins or combinations thereof) and/or the biosilica precursor or oligo/polysilicate has/have been encapsulated in a organic polymer, wherein the organic polymer preferably is poly(lactic acid).

In another embodiment of the invention the inventive injectable material is for the treatment of patients suffering from bone disorders, in particular osteoporosis, preferably for percutaneous injection into the fractured vertebra of osteoporotic patients by means of one or two bone biopsy needles (vertebroplasty and kyphoplasty).

In one embodiment the injectable material of the subject invention is for the formulation of an orally or parenterally-administrable drug for prophylaxis or therapy of bone disorders, in particular osteoporosis.

Preferably the injectable material of the present invention is formulated as a food supplement for prophylaxis or therapy of bone disorders, in particular osteoporosis, preferably as a supplement to yoghurt or other milk products for prophylaxis or therapy of bone disorders, in particular osteoporosis, wherein said material preferably is silica (biosilica), in particular polysilicate ("biosilicate") or nanoparticulate biosilica ("nanobiosilica"), or poly(lactic acid) encapsulated silica (biosilica). Specifically in combination with further resorbable additives, in particular polyphosphate, as a supplement to yoghurt or other milk products for prophylaxis or therapy of bone disorders, in particular osteoporosis.

### Detailed description of the Invention

This invention concerns the unexpected property of biosilica to increase the ratio of expressions of *osteoprotegerin* [*OPG*] and the *receptor activator for NF-KB ligand* [*RANKL*] in bone forming osteoblasts. The OPG : RANKL ratio has been shown as a key factor in the development of osteoporosis. The inventors demonstrate by quantitative real-time RT-PCR analysis and ELISA methods that biosilica, synthesized by the enzyme silicatein, causes a strong increase in OPG expression in osteoblasts (or osteoblast-like SaOS-2 cells), while the level of expression of *RANKL* remained unchanged. Furthermore, the inventors demonstrate that the synthesis of cartilaginous proteoglycans and sulfated glycosaminoglycans is down-regulated. Therefore, biosilica not only induces hydroxyapatite (HA) formation in osteoblasts as demonstrated in earlier studies of the inventors (EP 2005004738; US 11579020), but also stimulates OPG synthesis in osteoblasts (this invention) and thus counteracts those pathways that control RANKL expression and function, i.e. the maturation of pre-osteoclasts and the activation of osteoclasts. Based on this invention, biosilica is not only suitable for application in tissue engineering and for coating of medical implants (EP 2005004738; US 11579020), but also for application in prophylaxis/therapy of osteoporotic disorders. A further aspect of this invention is the description of various formulations of biosilica materials, based on the application of silicatein and/or the silicatein-interactor protein silintaphin-1, which can be administered either by percutaneous injection or used as a drug or food supplement. The discovery that biosilica unexpectedly acts as a modulator of the OPG : RANKL ratio which is crucial in the development of osteoporosis represents a significant progress beyond the state-of-the-art, allowing a rational prophylaxis and/or treatment of osteoporosis and related disorders. A further aspect of this invention is the finding that the silicatein/biosilica matrix up-regulates the expression of BMP2 (inducer of bone formation) in osteoblasts (osteoinductive potential of biosilica), whereas TRAP (modulator of bone resorption) expression remains unaffected.

In the experiments described below, osteoblast-like bone forming SaOS-2 cells have been used, which express the key proteins RANK, OPG, and RANKL, as well as BMP2.

### Biosilica material for prophylaxis or treatment of osteoporosis

This invention concerns an injectable material or a material to be used as a drug or food supplement, consisting of an enzyme or protein involved in biosilica (amorphous, hydrated silicon oxide) metabolism - or mixtures thereof - (silicatein or silintaphin-1 or silicatein-silintaphin-1 fusion proteins or combinations thereof) and provided with an N-terminal or C-terminal bound apatite (calcium phosphate)- or silica-binding oligocationic or oligoanionic tag, or a multiple thiol groups carrying tag.

The oligocationic or oligoanionic tag can consist of an oligo(amino acid), which can be composed of glutamic acid, aspartic acid or lysine or mixtures of these amino acids. Recombinant proteins (silicatein or silintaphin-1 or silicatein-silintaphin-1 fusion proteins) containing these tags can be synthesized according to state-of-the-art methods. Examples are described below. The oligo(amino acid) tag can have a length of six to ten amino acids, preferably 8 amino acids are used.

The Glu-tag, for example, allows for ionic interaction with Ca²⁺ ions of HA: Ca²⁺ ions of HA occur in two crystallographically different symmetry sites; four Ca²⁺ ions are arranged in columns while the remaining six Ca²⁺ ions form two triangular sets on the mirror planes. Binding of the Glu residues to the HA surface is based upon coordination/complex interactions between carboxyl groups and Ca²⁺ ions.

A further aspect of this invention concern a material containing a recombinant protein - or mixtures thereof - (silicatein or silintaphin-1 or silicatein-silintaphin-1 fusion proteins) with a multiple thiol groups carrying tag, preferably an oligo(cysteine) tag with 8 cysteine residues. Such a tag can bind iron ions which are required for the iron-dependent enzyme prolyl hydroxylase involved in collagen formation.

The enzyme or protein involved in biosilica metabolism can carry a tag on both its N-terminus and C-terminus, whereby the chemical nature of these two tags (oligocationic or oligoanionic tag, or multiple thiol groups carrying tag) can be either be different or the same.

The experiments described below describe a material, wherein a silicatein polypeptide or a silicatein fusion protein is used, that had been produced using a prokaryotic expression system. Also production using a eukaryotic expression system is possible.

Alternatively, the silicatein polypeptide or the silicatein fusion protein can be produced synthetically.

In addition, a silintaphin-1 polypeptide or a silintaphin-1 fusion protein can be used, that can also be produced using either a prokaryotic or a eukaryotic expression system.

Alternatively, a silintaphin-1 polypeptide or silintaphin-1 fusion protein can be used, that has been produced synthetically.

In the experiments described below, a silicatein polypeptide or a silintaphin-1 polypeptide from the demosponge *Suberites domuncula* is used.

Usually a silicatein polypeptide and/or a silintaphin-1 polypeptide is used, that had been purified and is essentially free from other proteins.

A further aspect of this invention concerns a material, characterized in that the tagged silicatein molecules or tagged silintaphin-1 molecules are bound via their protein tag to HA nanoparticles or HA - collagen nanoparticles.

The injectable material or the material used as a drug or food supplement can be modified ex vivo by biosilica formation using its silicatein or silicatein fusion protein component and a suitable biosilica precursor, consisting of water glass, orthosilicic acid, orthosilicates, monoalkoxysilanetriols, dialkoxysilanediols, trialkoxysilanols, tetraalkoxysilanes, alkyl- or aryl-silanetriols, alkyl- or aryl-silanediols, alkyl- or aryl-monoalkoxysilanediols, alkyl- or aryl-monoalkoxysilanols, alkyl- or aryl-dialkoxysilanols, alkyl- or aryl-trialkoxysilanes, or silicon-catecholate complexes.

For a schematic presentation of biosilica synthesis by HA bound Glu-tagged silicatein, achieved through addition of orthosilicate, see Figure 9C.

Furthermore, the injectable material or the material used as a drug or food supplement can be modified ex vivo by binding of nanoparticulate silica particles (nanosilica) or microparticulate silica particles or other metal oxide particles or mixtures thereof to its silintaphin-1 or silintaphin-1 fusion protein component.

Furthermore, the injectable material can be modified ex vivo by binding of iron [iron (II) and/or iron (III)] to the multiple thiol groups carrying tag. Iron ions are essential for prolyl hydroxylase activity, an enzyme involved in collagen synthesis. In addition, silica has been reported to stimulate the prolyl hydroxylase (Carlisle EM, Berger JW, Alpenfels WF. A silicon requirement for prolyl hydroxylase activity. Fed Proc 40:886, 1981).

Furthermore, the enzyme and/or protein components (silicatein or silintaphin-1 or silicatein-silintaphin-1 fusion proteins or combinations thereof), as well as the biosilica precursor can be encapsulated in an organic polymer such as poly(lactic acid) or poly(D,L-lactide)/polyvinyl pyrrolidone-based microspheres.

In addition, the injectable material or a material to be used as a drug or food supplement can be used in combination with polymethylmethacrylate (PMMA), HA, bioglass, collagen, polyphosphate or any other material used in therapy of bone disorders.

Moreover, only the polymeric inorganic component (biosilica) from the injectable material or the material used as a drug or food supplement can be applied after prior separation of the inorganic component from the organic component using state-of-the-art procedures.

The recombinant proteins (various isoforms of biocatalytically active silicatein and silintaphin-1, as well as the tagged silicatein or silintaphin-1 proteins and the silicatein-silintaphin-1 fusion proteins) can be expressed in a recombinant way, e.g., by *Escherichia coli* using a suitable state-of-the-art vector. cDNAs encoding various silicatein isoforms (e.g., silicatein-α and -β of the marine sponge *S*. *domuncula* and silicatein-α1 to silicatein-α4 of the freshwater sponge *Lubomirskia baicalensis)* have been used. The recombinant oligohistidine-tagged proteins can be purified using state-of-the-art technologies, e.g., by Ni-NTA affinity chromatography.

The present invention will now be further described in more detail in the form of preferred embodiments thereof in the following examples, nevertheless, without being limited thereto. For the purposes of the present invention, all references as cited herein are incorporated by reference in their entireties.

### Brief description of the drawings and sequences:

**Figure 1****.** Proposed effects of biosilica on osteoblasts, osteoclasts, and their progenitors. Biosilica enhances expression of OPG in osteoblasts. Osteoblasts have the potential to differentiate to HA-forming osteocytes and lining cells. OPG counteracts various effects of RANKL, a cytokine that induces preosteoclast maturation and osteoclast activation. Scheme.

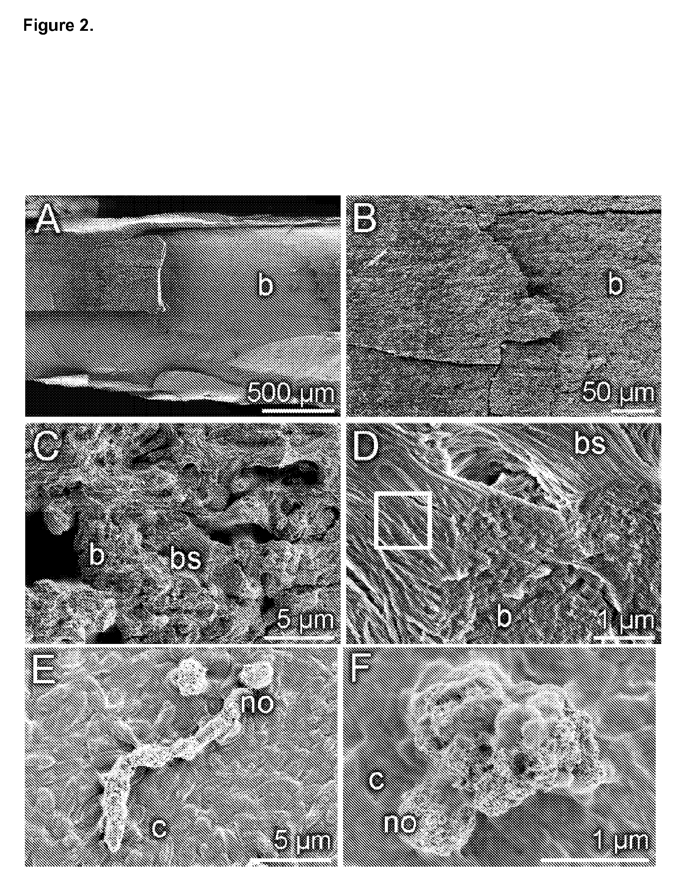

**Figure 2****.** Coating of bone HA with biosilica and mineralization of SaOS-2 cells. (**A-D**) Scanning electron microscopic analyses. (A and B) Uncoated bone HA with micro-and nano-structures at its surface (b). (C and D) Glu-tagged silicatein-α was immobilized on bone HA and then incubated with orthosilicate. This process elicited a smooth biosilica surface layer (bs). (**E, F**) Formation of HA nodules by SaOS-2 cells on bone HA. Cells were grown in McCoy's medium, supplemented with FCS, ascorbic acid, and ß-glycerophosphate. Cells (c) grown on Glu-tagged silicatein/biosilica coated bone HA formed HA nodules (no) during an incubation period of 5 days. The nodules are arranged longitudinally (E) or spherically (F).

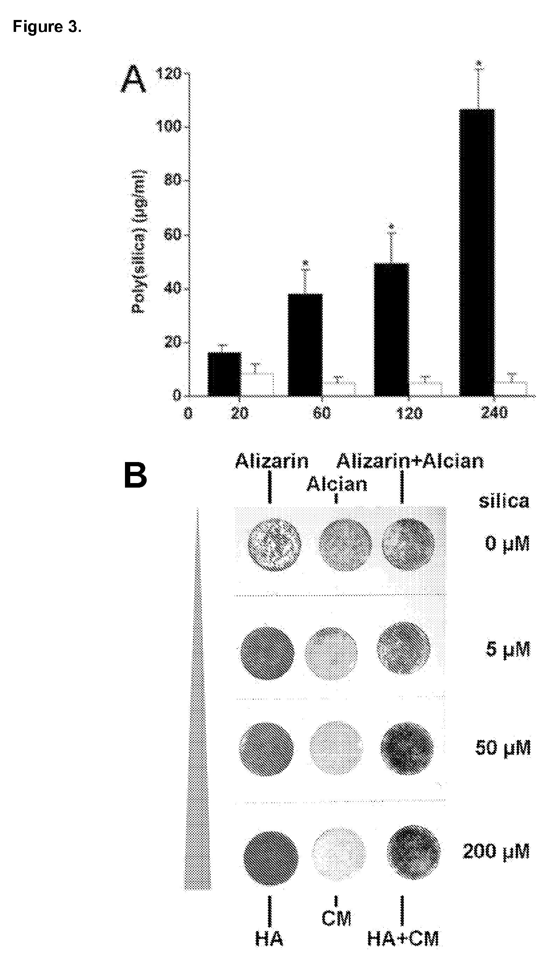

**Figure 3****.** Biosilica induced mineralization of SaOS-2 cells. **(A)** Quantification of silicatein-synthesized biosilica in culture dishes. Silicatein-coated culture wells had been incubated with 200 µM orthosilicate for 20-240 min. Then, poly(silicate) of aliquots (1 ml) was pelleted and quantified by the molybdenum blue colorimetric method (filled bars). As control, poly(silicate) formation in non-coated plates was quantified in parallel (open bars). **(B)** Biosilica induced mineralization of osteoblast-like SaOS-2 cells. Cover slips within 24-well plates were coated with recombinant silicatein and, then, incubated with orthosilicate-free Tris-HCl buffer (0 µm silica; control) or with increasing concentrations of orthosilicate (5-200 µM, 4 h). Subsequently, SaOS-2 cells were seeded in McCoy's medium (including ascorbic acid and ß-glycerophosphate) and incubated for 7 days. Finally, the slips were removed and stained with Alizarin Red S (for HA), Alcian Blue (for cartilageous material [CM]), or a combination of both dyes.

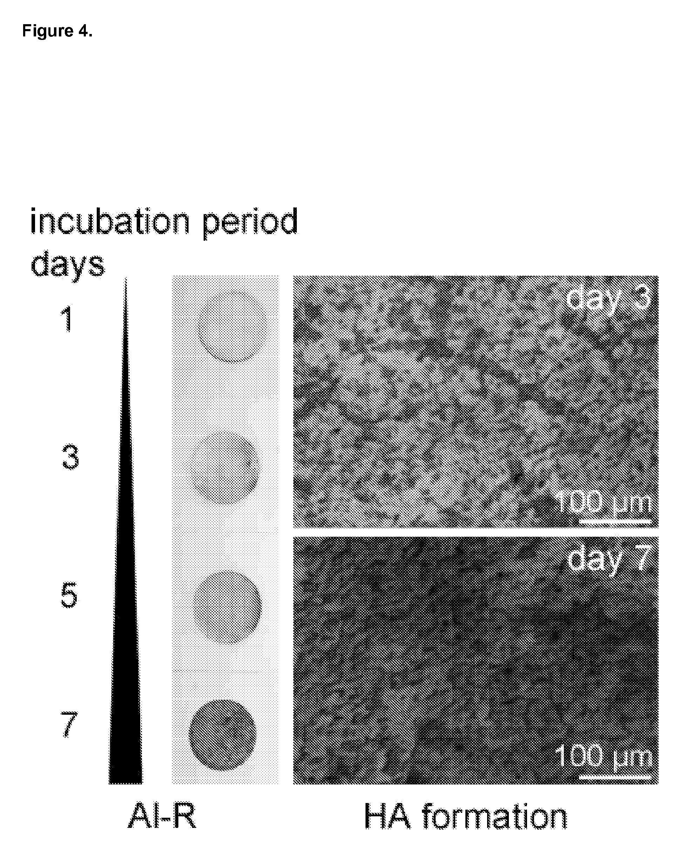

**Figure 4****.** HA formation of SaOS-2 cells, cultivated on Glu-tagged silicatein/biosilica coated Ca-P cover slips. Cells were cultivated for 1 to 7 days in McCoy's medium (containing ascorbic acid and ß-glycerophosphate), as indicated (left panel). The discs on which the cells had been grown for 1 to 7 days were stained with Alizarin Red S (Al-R) to assess the extent of HA formation. The right panel depicts cells that were stained after 3 (upper image) or 7 days culturing (lower image), visualized by reflected light microscopy.

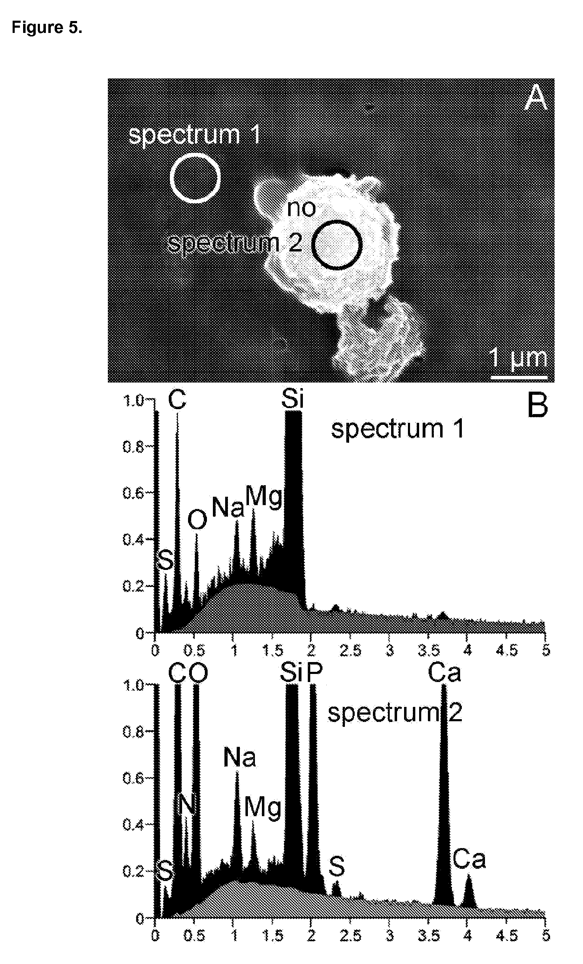

**Figure 5****.** SEM and EDX analysis of HA nodules of SaOS-2 cells. Cells were grown in mineralization medium for 7 days on a Glu-tagged silicatein/biosilica coated Ca-P cover slips. Then, one specimen was inspected by SEM (**A**). In this image, the two areas analyzed by EDX are indicated by circles. Spectrum 1: cell area; spectrum 2, nodule (no). (**B**) Corresponding EDX analysis. Spectrum 1, spectrum of cells. Spectrum 2, spectrum of nodule. Grey areas represent the spectra originating from the Bremsstrahlen.

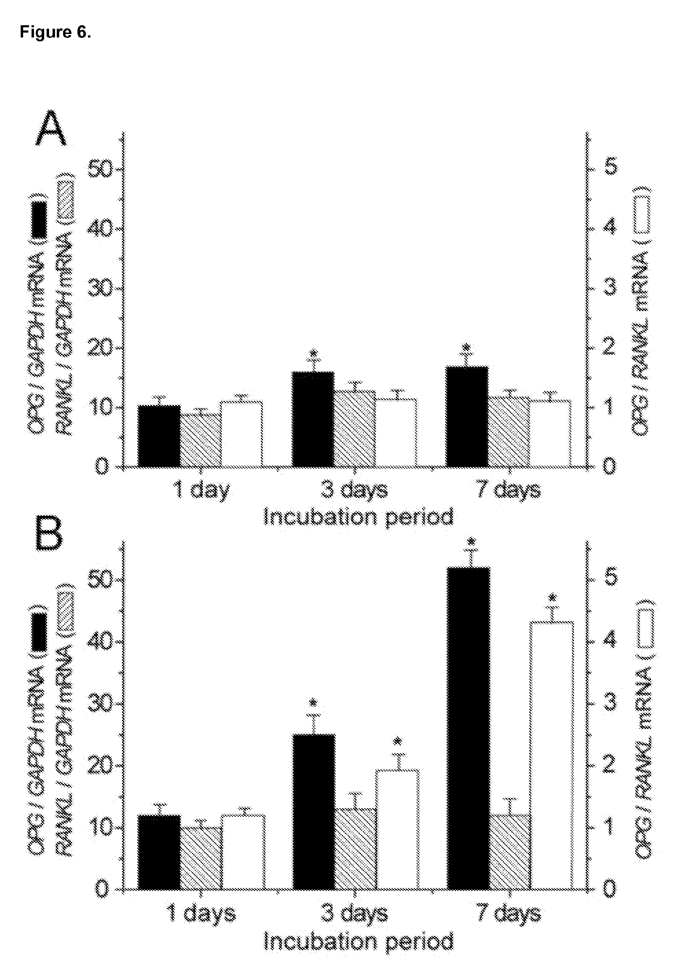

**Figure 6**. Biosilica regulated transcription of OPG and *RANKL* in SaOS-2 cells. RNA was extracted from cells that had been cultivated for 1, 3, or 7 days in silicatein-coated (**A**) or silicatein/biosilica-coated (**B**) well plates. Then, qRT-PCR was used to determine expression levels of OPG, *RANKL,* and *GAPDH.* The latter one was used as reference for normalization. Closed bars, ratio of *OPG*/*GAPDH* expression; hatched bars, ratio of *RANKL*/*GAPDH* expression; open bars, ratio of normalized *OPG*/*RANKL* expression. Standard errors of the means (SEM) are indicated (n = five experiments per time point); P < 0.05.

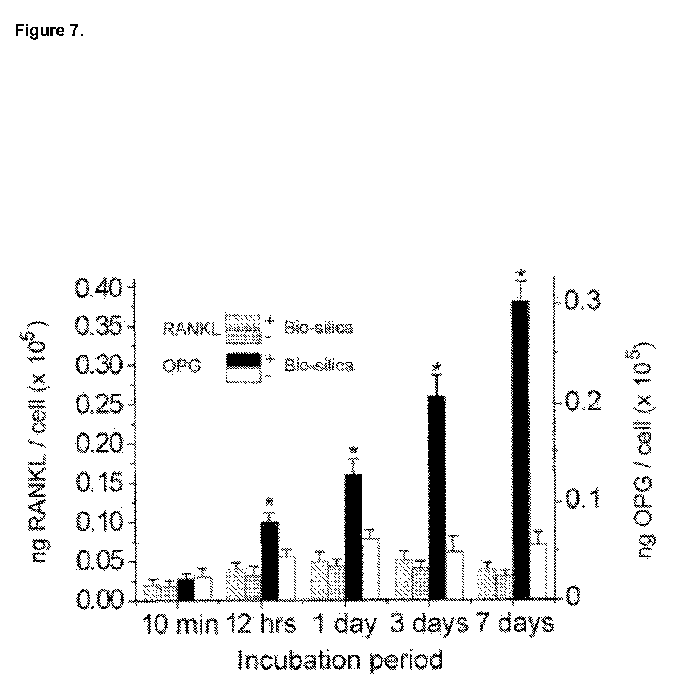

**Figure 7**. Biosilica regulated OPG and RANKL protein expression in SaOS-2 cells. SaOS-2 cells were grown in silicatein-coated (- biosilica) or silicatein/biosilica-coated (+ biosilica) well plates. After the indicated time, protein concentrations of RANKL and OPG in the culture medium were determined by ELISA. SD values are indicated; P < 0.05.

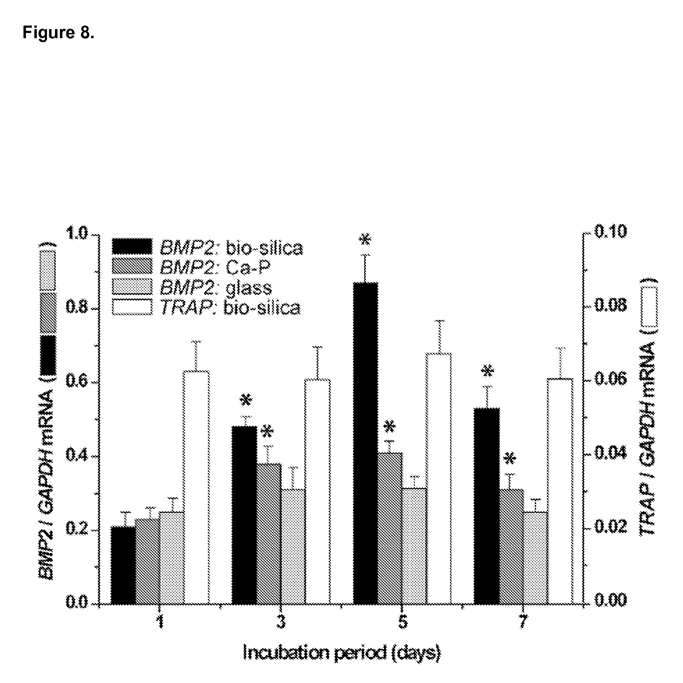

**Figure 8**. Differential expression of *BMP2* and *TRAP* in SaOS-2 cells grown on different matrices for the indicated time. The technique of qRT-PCR was used to determine expression levels of *BMP2, TRAP,* and *GAPDH* (housekeeping gene; used as reference for normalization). Filled bars represent *BMP2* expression of cells grown on Glu-tagged silicatein/biosilica coated Ca-P cover slips (black bars), on uncoated Ca-P cover slips (dark grey), or on glass cover slips (light grey). Open bars (white) represent *TRAP* expression of cells that had been cultivated on biosilica coated Ca-P cover slips. Standard errors of the means (SEM) are shown (n = five experiments per time point); P < 0.05 indicated by asterisks.
**Figure 9****.** Application of the materials according to the invention for treatment of vertebral fractures (compression fractures) of osteoporotic patients. (**A**) Application in vertebroplasty: The biosilica-based material (comprising biosilica formed ex vivo or tagged silicatein/silintaphin-1/silicatein-silintaphin-1 fusion protein administered together with a suitable biosilica precursor) is injected into the fractured vertebra either alone or together with some other material used in vertebroplasty such as polymethylmethacrylate (PMMA) by a bone biopsy needle (percutaneous injection). (**B**) Application in kyphoplasty: A balloon (bone tamp) is inserted into the vertebral body by means of two bone biopsy needles under image guidance. When inflated with radiocontrast medium and the biosilica-based material (see above) either alone or together with some other material used in vertebroplasty such as polymethylmethacrylate (PMMA), the bone tamp re-expands the vertebral body (elevation of its endplates). The restructured vertebral body is stabilized by the polymeric material (biosilica alone or biosilica and PMMA) after hardening. Recruitment of osteoblasts and restoration of the balance between bone formation (osteoblasts) and degradation (osteoclasts) by the osteoinductive activity of the biosilica material according to the invention finally result in replacement of the injected material by body's own bone material. (**C**) Schematic outline of biosilica formation by Glu-tagged silicatein in the presence of substrate. Silicatein binds via its high-affinity Glu-tag to the trabeculae. Following its immobilization on the HA substrate (trabeculae), Glu-tagged recombinant silicatein facilitates biosilica synthesis in the presence of a silica source (here, orthosilicate). The matrix (trabeculae) thus modified is bioactive and consequently enhances HA formation and OPG expression by mineralizing cells (osteoblasts; here, experimentally, SaOS-2 cells, following addition of ß-glycerophosphate).
**SEQ ID No. 1:** Amino acid sequence of the silicatein-α polypeptide from *Suberites domuncula.*
**SEQ ID No. 2:** Nucleic acid sequence of the cDNA coding for the silicatein-α polypeptide from *Suberites domuncula.*
**SEQ ID No. 3:** Amino acid sequence of the silintaphin-1 polypeptide from *Suberites domuncula.*
**SEQ ID No. 4:** Nucleic acid sequence of the cDNA coding for the silintaphin-1 polypeptide from *Suberites domuncula.*

### Examples

Figure 1 shows a schematic presentation of the *in vivo* regulatory role of biosilica according to this invention on the expression/release of OPG in osteoblasts. Biosilica increases the expression of OPG, whereas the expression of RANKL is not affected. By binding to OPG (increased in the presence of biosilica according to this invention), RANKL is sequestered and unavailable to bind to its (pre)ostecloastic receptor, RANK. Therefore, biosilica negatively affects, via consequential secondary effects, pre-osteoclast maturation and osteoclast activation. Hence, biosilica shows a considerable biomedical potential in therapy and prophylaxis of osteoporosis.

This conclusion is based on the following results underlying this invention.

### Growth of osteoblasts in the presence of orthosilicate and toxicity

The potential toxicity of orthosilicate and its effect on cell growth was tested *in vitro* with osteoblast-like SaOS-2 cells. SaOS-2 cells were exposed to orthosilicate (obtained by pre-hydrolyzation of tetraethoxysilane, TEOS). After 72 h, cell density and viability were assessed by XTT assay and trypan-blue exclusion test, resp. Addition of orthosilicate resulted in increased absorbance values, indicating higher cell concentrations (XTT assay). Maximal values are seen at 100 µM with 170% with respect to the control. Application of trypan blue revealed that after 72 h, independent of the orthosilicate concentration applied, the number of trypan blue excluding cells (living cells) never dropped below 94%. Therefore, orthosilicate (1) has a cell growth-stimulating effect (XTT assay) and (2) does not show cell toxicity (trypan-blue-based viability assay).

### Coating of bone HA with silicatein/biosilicate

In the experiment shown in Figure 2A-D, bone HA was incubated with recombinant Glu-tagged silicatein-a and then with 200 µM orthosilicate. Non-modified HA samples display micro- and nanoporous structured surfaces (Figure 2A and B). However, after immobilization of Glu-tagged silicatein-α and subsequent incubation with orthosilicate, a smooth layer of nanoscale biosilica was formed on the HA surface (50-150 nm thicknes; Figure 2C and D). EDX analyses of the coated HA showed strong signals of Si and O, originating from the biosilica layer, while weaker signals of Ca and P were caused by bone HA. EDX spectra of uncoated bone material lack the Si peak.

### Preparation of biosilica in silicatein-coated culture plates

Recombinant silicatein was immobilized on cover slips or bottoms of multi-well plates (culture plates). After adsorption (3 h), immobilization of silicatein was verified immunologically by using polyclonal anti-silicatein antibodies.

Colorimetric quantification using a molybdenum blue-based assay revealed a time-dependant formation of up to 105 g biosilica per ml reaction (Figure 3A, filled bars). In controls (uncoated plates), maximally 10 µg/ml poly(silicate) were detected (Figure 3A, open bars).

Biosilica was also detected through Rhodamine 123 staining and fluorescence microscopy.

In addition, the siliceous aggregates were analyzed by EDX. The spectra displayed two major peaks, corresponding to O and Si.

The particles formed during the initial phase of biosilica synthesis (20-60 min) were examined by TEM; they were spherical, with an average size of 50 to 60 nm. With longer time the particle shapes became more bulky and the sizes increased.

### Effect of biosilica on mineralization of osteoblasts

The effect of biosilica on mineralization activity of osteoblast-like SaOS-2 cells was investigated, using Alizarin Red S and Alcian Blue to evaluate HA deposition and cartilaginous proteoglycans/sulfated glycosaminoglycans formation, respectively.

In the experiment shown in Figure 3B, silicatein was immobilized on cover slips that were subsequently incubated with 0-200 µM orthosilicate for 4 h. During that time biosilica formation was dependent of the orthosilicate concentration, 8±3 µg/ml (5 µM orthosilicate), 32±7 µg/ml (50 µM), or 81±12 µg/ml (200 µM), as determined by the "Silicon Test Colorimetric Assay Kit". Then, the coated slips were placed into 24-well plates, overlaid with SaOS-2 cells. After 7 days, the slips were stained with Alizarin Red S, or Alcian Blue, or simultaneously with both dyes. Figure 3B shows only a faint orange-red staining of the cells with Alizarin Red S [staining for HA] that had been grown on non-coated slips (0 µm silica). However, the biosilica-coated samples revealed a considerable staining, whose intensity correlated to the orthosilicate concentration initially applied. Thus, 200 µM orthosilicate elicited a homogenous red color, reflecting an increased HA formation by the cells. In contrast, in samples that had been stained with Alcian Blue (staining for cartilaginous proteoglycans and sulfated glycosaminoglycans), the color intensity inversely correlated to increasing orthosilicate concentrations. Thus, samples that had been exposed to 200 µM orthosilicate appeared light blue whereas control samples (0 µM silica) stained dark blue. In addition, double-staining of the samples with both dyes elicited a dark red/blue color.

The observed biosilica-stimulated increase in mineralization and the decrease in cartilaginous molecules are favorable with respect to the treatment of osteoporosis, since cartilage damage is inversely correlated with the bone mineral density index.

In a further set of experiments, SaOS-2 cells were grown on sections of bone HA that had been coated with Glu-tag-silicatein/biosilica or remained uncoated (control). To stimulate mineralization, cells were cultivated in medium containing ascorbic acid and ß-glycerophosphate. After 5 days, control cells revealed only few signs of mineralization, i.e. occasional clusters of small HA rods, ca. 20-50 nm in size. In contrast, cells grown on silicatein/biosilica coated HA frequently showed clusters of HA rods and nodules (Figure 3E,F). Nodules of > 1 µm in size emerged from cell clusters, consisting of 3-8 single cells. At times, the HA nodules were organized in longitudinal arrays (Figure 3E) or spherical spots (Figure 3F).

To quantify HA formation, SaOS-2 cells were cultivated in McCoy's medium (containing ascorbic acid and ß-glycerophosphate) for 1-7 days on Ca-P cover slips that had been coated with silicatein/biosilica or remained uncoated (control). As a further control, cells were seeded on plain glass cover slips. Subsequently, HA formed was stained with Alizarin Red S. After incubation of SaOS-2 cells on biosilica coated Ca-P cover slips for 1 day, only a faint staining was seen that, however, progressively intensified to dark red during prolonged cultivation until day 7 (Figure 4, left panel). Concurrent microscopic inspection revealed a heterogeneous staining after 3 days that became denser after 7 days (Figure 4, right panel).

Furthermore, EDX analyses were performed to assess the elemental composition of the nodules formed by SaOS-2 cells. Following electron microscopic inspection (Figure 5A), two spectra were recorded, focusing either on the cells in the nodule's vicinity (Figure 5B [spectrum 1]; control) or directly on one nodule (Figure 5B [spectrum 2]). In the former spectrum, Ca or P was not detected but Si, O, C, Mg and Na, originating from both cells and biosilica coating of the substrate. In the latter spectrum, additional peaks of Ca and P were detected, indicating the presence of HA.

To quantify HA formation, Alizarin Red S stained cells were harvested. Following spectroscopic measurement of optical density, the amount of Alizarin Red S bound to cells was normalized to the total amount of DNA in order to allow a comparison between cultures and assays. Samples where the cells had been grown on plain glass cover slips showed only low amounts of HA. At day 1 the amount of bound Alizarin Red S was as low as 0.06 nmole/µg DNA and increased to 0.26 nmole/µg DNA after 7 days. Similarly, after growth of SaOS-2 on Ca-P cover slips, 0.07 nmole/µg DNA was calculated after the first day. However, after 7 days, 0.32 nmole/µg DNA was detected. Furthermore, cells grown on silicatein/biosilica coated Ca-P cover slips elicited an even higher increase of values from 0.08 nmole/µg DNA (day 1) to 0.42 nmole/µg DNA (day 7).

### Effect of biosilica on transcription of OPG and RANKL in osteoblasts

In the following experiments, the expression of OPG and RANKL (transcript level and protein level) was studied in osteoblast-like SaOS-2 cells. Similar results can also be obtained with other bone forming cells.

The expression levels of OPG and *RANKL* was determined by qRT-PCR using transcription of the housekeeping gene *GAPDH* as reference. Figure 6 shows the results for SaOS-2 cells that had been cultivated for 1, 3, or 7 days on silicatein-coated (control) or on silicatein/biosilica-coated matrices. Whereas in control samples the expression of both OPG and *RANKL* changed only unsignificantly during day 1 to 7 (Figure 6A), a strong increase of OPG expression was observed during day 3-7 upon incubation on the silicatein/biosilica-coated matrix (4.8-fold after day 7, compared to day 1) (Figure 6B). During this time RANKL expression did not change markedly. Hence, the ratio of *OPG*/*RANKL* expression increased considerably from 1.3 (day 1) to 4.3 (day 7) (Figure 6B).

### Effect of biosilica on OPG and RANKL protein expression in osteoblasts

To assess the biosilica stimulated differential expression of OPG and *RANKL* on the protein level, an ELISA system was employed. In the experiment shown in Figure 7 concentrations of both cytokines were determined in the culture supernatants and correlated to the cell number, following incubation of SaOS-2 cells for maximal 7 days in uncoated or biosilica-coated wells. At the beginning of the incubation (after 10 min) the expression of both, RANKL and OPG was low, irrespectively whether the cells are grown on a biosilica-coated matrix or not. During the subsequent 7 days, the concentration of RANKL in the medium did not change markedly. In contrast, in the presence of biosilica the OPG level significantly increased. The control samples did not show a significant change of the OPG level during that time.

Therefore, biosilica induces not only an up-regulation of OPG-expression but also an increased release of OPG into the culture medium. In contrast, expression of RANKL remains unchanged.

### Effect of biosilica on BMP2 and TRAP expression in osteoblasts

In the following experiments, the expression of the two further marker genes, *BMP2* and *TRAP,* was assessed during proliferation and differentiation of SaOS-2 cells growing on the biosilica substrate. SaOS-2 cells were incubated in mineralization medium (McCoy's medium/ascorbic acid/ß-glycerophosphate) for 1-7 days, either on Glu-tag-silicatein/biosilica coated Ca-P cover slips, untreated Ca-P cover slips, or glass cover slips. RNA was extracted and subjected to qRT-PCR analysis. Expression of the house keeping gene *GAPDH* was used as reference. The respective expression levels of *BMP2* and *TRAP* at day 1, after transfer of the cells into the mineralization medium, were taken as basis. As summarized in Figure 8, *BMP2* expression strongly increased in the cultures grown on biosilica coated slips, from 2.3-fold (3 days) to 3.8-fold (5 days) (Figure 8). After 7 days, the expression level decreased to 2.5-fold. In comparison, *BMP2* expression of SaOS-2 cells grown on uncoated Ca-P cover slips was much lower. In contrast, the expression of *BMP2* did not significantly change in cells that had been grown on plain glass cover slips.

In parallel, *TRAP* transcription was evaluated by qRT-PCR. As shown in Figure 8, no change of *TRAP* expression was detected, irrespective of the culture conditions used.

### Application of the materials for treatment of osteoporosis

The biosilica material and its various formulations according to this invention can be applied for treatment of patients suffering from osteoporosis (postmenopausal osteoporosis and osteoporosis of the elderly).

Based on its activity to induce increased OPG levels, the biosilica material and its various formulations according to this invention may also act as a therapeutic agent in other disorders characterized by increased resorption, such as rheumatoid arthritis, Paget's disease, periodontitis, carcinomatosis of bone, haematologic disorders (multiple myeloma), hypercalcaemic syndrome, or disorders with locally increased resorption.

Because of the role of the OPG/RANKL/RANK system in secondary skeletal-related and vascular disorders, this invention may also be significant in prophylaxis/therapy of further diseases including artherosclerosis, diabetes, and metastases.

### Application for percutaneous injection (vertebroplasty and kyphoplasty)

The materials described by the inventors can be used for percutaneous injection into the fractured vertebra of osteoporotic patients by means of one or two bone biopsy needles (vertebroplasty and kyphoplasty).

A schematic presentation of the application of the materials for treatment of vertebral fractures (compression fractures) of osteoporotic patients is shown in Figure 9. Figure 9A shows the application in vertebroplasty. The material, comprising either biosilica formed ex vivo or tagged silicatein/silintaphin-1/silicatein-silintaphin-1 fusion protein administered together with a suitable biosilica precursor, is injected into the fractured vertebra either alone or together with some other material used in vertebroplasty such as polymethylmethacrylate (PMMA). Figure 9B shows the application in kyphoplasty. A balloon (bone tamp) is inserted into the vertebral body by means of two bone biopsy needles under image guidance. When inflated, the bone tamp re-expands the vertebral body. The restructured vertebral body is then stabilized by the injected polymeric material (biosilica alone or biosilica and PMMA) after hardening. Recruitment of osteoblasts and restoration of the balance between bone formation (osteoblasts) and degradation (osteoclasts) by the modulatory effect of the biosilica material according to the invention on the RANK/RANKL/OPG system and its osteoinductive activity finally result in replacement of the injected material by body's own bone material. Figure 9C shows a schematic presentation of biosilica formation by the injected Glu-tagged silicatein in the presence of substrate. Silicatein binds via its Glu-tag to the trabeculae. Alternatively silicatein molecules containing another oligoanionic or oligocationic tag such as an oligo-Asp tag or an oligo-Lys tag can be used. Following its immobilization on the trabeculae (HA), silicatein facilitates biosilica synthesis in the presence of a suitable silica precursor (for example: orthosilicate or water glass injected together with the tagged silicatein molecules). The biosilica coating of the trabeculae induces a recruitment of osteoblast and increased mineralization (HA formation) by these cells. In addition, it induces OPG expression, resulting in decreased bone-resorbing osteoclast activity due to capturing of RANKL by OPG released from osteoblasts.

The efficiency of therapy can be demonstrated, for example, by determination of microcallus formation or binding of nanosilica to collagen.

It should be noted that silicatein has been demonstrated to be non toxic in animal experiments.

### Application as orally or parenterally-administrable drug for prophylaxis or therapy of osteoporosis

The materials described by the inventors can also be used for the formulation of an orally or parenterally-administrable drug for prophylaxis or therapy of osteoporosis and other bone disorders. The material can be encapsulated in poly(lactic acid) or poly(D,L-lactide)/polyvinyl pyrrolidone-based microspheres or administered in a different form, according to state-of-the-art techniques.

### Application as a food supplement for prophylaxis or therapy of osteoporosis

Biosilica-based materials can be administered as a food additive (supplement) for prophylaxis or therapy of osteoporosis and other bone disorders. Dietary silicon is known to enter and to leave the body via the gastrointestinal tract and urinary excretion, respectively (reviewed in: Jugdaohsingh R. Silicon and bone health. J Nutr Health Aging 11:99-110, 2007). Polymeric silica (polysilicate or silica nanoparticles ["nanobiosilica"]), given either alone or in combination with other resorbable food additives such as polyphosphate according to this invention, can increase the bioavailability of silicon. Biosilica is composed of nanoparticles of a diameter of 50 to 70 nm (Tahir MN, Théato P, Mutter WEG, Schröder HC, Janshoff A, Zhang J, Huth J, Tremel W. Monitoring the formation of biosilica catalysed by histidin-tagged silicatein. ChemComm 24:2848-2849, 2004). Particles of that size are readily taken up by cells through endocytosis (Jin H, Heller DA, Sharma R, Strano MS. Size-dependent cellular uptake and expulsion of single-walled carbon nanotubes: single particle tracking and a generic uptake model for nanoparticles. Nano 3:149-158, 2009). Uptake of biosilica nanoparticles (nanobiosilica) will increase the available concentration of silica, being of advantage compared to other formalations based on orthosilicate or other monomeric silica derivatives.

### Application as a supplement to milk products for prophylaxis or therapy of osteoporosis

Biosilica-based materials, in particular polysilicate ("biosilicate") and nanoparticulate biosilica ("nanobiosilica") can be applied as a supplement to yoghurt or other milk products for prophylaxis or therapy of bone disorders, in particular osteoporosis.

### Application of encapsulated biosilica

The biosilica material, in particular polysilicate ("biosilicate") and nanoparticulate biosilica ("nanobiosilica") or water glass, can be encapsulated in poly(lactic acid) or poly(D,L-lactide)/polyvinyl pyrrolidone-based microspheres or other nontoxic polymers using state-of-the art procedures and given as a supplement to yoghurt or other milk products for prophylaxis or therapy of osteoporosis and other bone disorders.

### Application in combination with further resorbable additives

The biosilica material, in particular polysilicate ("biosilicate") and nanoparticulate biosilica ("nanobiosilica") or water glass, can be applied as a supplement to yoghurt or other milk products in combination with further resorbable additives, in particular polyphosphate, and used as food supplement for prophylaxis or therapy of osteoporosis and other bone disorders.

### Methodology

### Expression and isolation of recombinant silicatein and Glu-tagged silicatein

The preparation of the recombinant silicatein is preferentially performed in *E. coli.* However, expression of the recombinant protein in yeast and mammalian cells is also possible and has been successfully performed. In short, S. *domuncula* silicatein-α cDNA (NCBI accession number CAC03737.1), encoding the mature silicatein segment from amino acid [aa] aa₁₁₃ to aa₃₃₀, is ligated into a suitable expression vector, for example pQ30 A (Qiagen) via *Bam*HI (5'-end) and *Hind*III (3'-end) restriction sites. *E. coli* host strain M15 (Qiagen) is transformed with the resulting construct and cultured in LB medium, supplemented with 50 µg/ml carbenicillin (AppliChem) at 37°C. The expression of the His-tagged fusion protein is induced by 1 µM isopropyl β-D-thiogalactopyranoside during overnight cultivation at 37°C. Then, bacteria are harvested by centrifugation and the resulting cell pellet is resuspended in IB Solubilization Reagent (Pierce). Following centrifugation, the clear supernatant is purified by affinity chromatography, for example by using the PROFINIA system (Bio-Rad). In order to facilitate refolding of recombinant silicatein, the eluted protein is diluted in a suitable refolding buffer (for example: 50 mM Tris-HCl, pH 8.5; 0.5 M L-arginine, L-glutathione [9 mM GSH, 1 mM GSSH], 0.3 M NaCl, 1 mM KCI). Finally, the samples are dialyzed against a Tris/HCl buffer (pH 8.2; 50 mM Tris-HCl, 24 mM NaCl).

Recombinant glutamic acid (Glu)-tagged silicatein-α can be prepared as follows. A pBAD/glll A construct or another suitable construct can be used, which encodes mature silicatein, ligated in frame with an N-terminal sequence, encoding 8 Glu residues (Glu-tag), and a C-terminal sequence, encoding 6 His residues (His-tag).

Whereas the Glu-tag confers binding affinity to HA, the His-tag is required for metal affinity chromatography of the recombinant protein. The resulting construct can be cloned in bacteria, yeast, or mammalian systems. For example, following transformation of TOP10 *E*. *coli* cells (Invitrogen), recombinant protein expression is induced by arabinose (0.2%) for 24 h. Subsequently, proteins are extracted and purified by Ni²⁺-NTA affinity chromatography. To induce refolding, the recombinant protein is dialyzed against one hundred volumes of a suitable refolding buffer (for example: 0.7 M L-arginine, 5.0 mM EDTA, 0.1 % [v/v] CHAPS, 10 mM glutathione, 1.0 mM glutathione disulfide, pH 7.2) and one thousand volumes of storage buffer (1 x phosphate-buffered saline [PBS], supplemented with 300 mM NaCl, 70 mM L-arginine, 1.0 mM EDTA, and 7% [v/v] glycerol, pH 7.2).

### Expression and isolation of recombinant silintaphin-1 and Glu-tagged silintaphin-1

The preparation of the recombinant silintaphin-1 is preferentially performed in *E*. *coli.* However, expression of the recombinant protein in yeast and mammalian cells is also possible and has been successfully performed. The combination of an appropriate forward primer, for example: 5-TCA TCA GAA GAG ACC CCA GTA GA-3, and of an appropriate reverse primer, for example: 5-GTC TTC TGC TTT TGT CTC CTC A-3 is used to amplify the silintaphin-1 ORF (nt₁₀₇₋₁₂₆₁), excluding Mₛₜₐᵣₜ and stop codon. The amplicon is inserted into a suitable expression vector, for example: pTrcHis2-TOPO (Invitrogen), in frame with an N-terminal Mₛₜₐᵣₜ and a C-terminal 6xHis-tag. Following transformation of BL21 cells the recombinant protein can be purified, e.g., by using the histidine-tag, on suitable affinity matrices, such as Ni-NTA, under native conditions (Qiagen, Hilden, Germany). Protein concentrations can be measured with commercial kits, for example the "2-D Quant Kit" (GE Healthcare). Subsequently, the protein is analyzed by SDS-PAGE. After blotting on PVDF membranes (Millipore), the recombinant protein is detected with anti-histidine antibodies (Qiagen).

Recombinant Glu-tagged silintaphin-1 can be prepared as follows. The tag can comprise, e.g., 8-10 Glu residues, attached N-terminal or C-terminal to the protein. Accordingly, the aforementioned primers are adapted, e.g., the foreward primer sequence 5-GAA GAG GAA GAG GAA GAG GAA GAG TCA TCA GAA GAG ACC CCA GTA GA-3, carries 8 additional 5'-terminal triplets (underlined) in frame, each one coding for Glu. After transfer into an appropriate expression vector, the resulting construct can be cloned in bacteria, yeast, or mammalian systems. Following induced expression the protein is purified as described above.

### Isolation and purification of silicatein and silintaphin-1 using antibodies

Silicatein and silintaphin-1 can be further purified on an affinity matrix. The affinity matrix can be prepared, for example, by immobilization of a silicatein- or silintaphin-1-specific antibody on a solid phase (CNBr-activated Sepharose or another suitable carrier). Monoclonal or polyclonal antibodies against silicatein or silintaphin-1 can be used, which are prepared following standard methods (Osterman (1984) Methods of Protein and Nucleic Acid Research Vol. 2; Springer-Verlag [Berlin]). Coupling of the antibody to the matrix is performed according to the instructions of the manufacturer. Elution of purified silicatein or silintaphin-1 is performed by pH change or change in ionic strength.

### Biosilica synthesis by immobilized Glu-tagged silicatein

Bone slices and Ca-P coated Osteologic cover slips were incubated in 24-well plates with refolded Glu-tagged silicatein (370 µg/ml PBS) for 6 h (20°C) in a final volume of 2 ml. Then, the plates were washed with 50 mM Tris-HCl buffer (pH 7.4; 150 mM NaCl). The binding of recombinant silicatein was assessed by immunodection, using polyclonal anti-silicatein antibodies. Then, slices and cover slips were incubated for 6 h (20°C) with a solution of 200 µM orthosilicate (prehydrolyzed TEOS [5 mM TEOS were mixed in a 1:3 molar ratio with 1 mM HCl for 30 min and subsequently neutralized]) in 50 mM Tris-HCl (pH 7.4). The final volume was 300 µl/well. Using this procedure, 57±11 µg of biosilica in the assays with bone slices [53±14 µg biosilica in the assays with cover slips] were synthesized in each well of the 24-well plates, as determined by the "Silicon Test Colorimetric Assay Kit". As controls, either uncoated bone slices, Ca-P coated cover slips without immobilized enzyme, or plain glass cover slips (12 mm; Bioscience tools) were used.

### Cells and incubation conditions

SaOS-2 cells (human osteogenic sarcoma cells) were cultured in McCoy's medium, supplemented with 5% heat-inactivated FCS [fetal calf serum], 2 mM L-glutamine, and gentamicin (50 µg/ml) in 25 cm² flasks or in multi-well plates (Orange Scientifique) in a humidified incubator at 37°C and 5% CO₂. Routinely, 3 x 10⁵ cells were added per well (3 ml total volume). For the experiments the cells were cultivated either on untreated substrates or on substrates that had been coated with silicatein/biosilica (see below).

To avoid potential effects of fresh serum on gene expression, medium with 5% FCS was used 2 days before expression analyses were initiated.

For the experiments with Ca-P coated cover slips or bone slices, cells were seeded onto Ca-P coated cover slips (BioCoat Osteologic cover slips) that had been designed to cultivate osteoblast and osteoclast cells *in vitro.* Alternatively, the cells were grown on surfaces of bone sections of rabbit femora (100 µm thickness). The slices were washed with PBS and incubated in 100 mM Tris-HCl buffer (pH 7.4; 6 M guanidine-HCl) for 72 h (4°C). Subsequently, they were successively treated with 5% hydrogen peroxide (60 min; 4°C) and 1.2 M HCl (20 min; 4°C) to remove all organic material. Finally, the slices were washed in PBS for 12 h.

Routinely, 5 x 10⁴ SaOS-2 cells were seeded onto bone sections or Osteologic cover slips in 24-well plates, in a final volume of 2.5 ml. To investigate HA formation, the SaOS-2 cells were incubated with McCoy's medium that had been supplemented with 15% FCS, 50 µM ascorbic acid, and 5 mM ß-glycerophosphate.

### Cell proliferation/viability assays

Orthosilicate can be prepared from prehydrolyzed TEOS as follows. A stock solution of 5 mM TEOS is mixed in a 1:3 molar ratio with 1 mM HCl for 30 min and subsequently neutralized. The orthosilicate formed is added to the cells at 10-1000 µM. SaOS-2 cells are seeded at a density of 5x10³ cells per well of a 96-multiwell plate (Orange Scientifique) and cultured for 72 h in McCoy's medium, supplemented with 5% FCS in the absence or presence of orthosilicate. Then, cell proliferation is determined by a colorimetric method based on the tetrazolium salt XTT (Cell Proliferation Kit II), following the recommendations of the supplier. Absorbance of the samples is determined with a spectrophotometer (ELISA reader) at 540 nm and, as reference wavelength, at 630 nm. In a second approach, cell viability was quantified by the trypan-blue exclusion assay. For this experiment, SaOS-2 cells are seeded at a density of 15x10³ cells per well of a 24-multiwell plate (Orange Scientifique) and cultured in McCoy's medium with 5% FCS in the absence or presence of orthosilicate. The number of viable and dead cells is determined microscopically after 72 h.

### Silicatein-coating of culture wells

To coat 6-well plates with recombinant silicatein, the plates are incubated with 200 µl/cm² of a solution containing 5 µg/ml of the recombinant enzyme in 50 mM Tris-HCl (pH 7.4) and 150 mM NaCl. After incubation for 3 h at 20°C, the plates are washed once with the Tris-HCl buffer. Then, to evaluate immobilization of silicatein, the wells are washed with PBS (pH 7.4) and blocked with 3% bovine serum albumin [BSA] for 1 hr at room temperature. Afterwards, the wells are incubated consecutively with polyclonal rabbit anti-silicatein antibodies (1:5,000 dilution in 0.3% BSA) and anti-rabbit IgG (alkaline phosphatase [AP]-conjugated secondary antibodies). After a final washing step, bound immunocomplexes are visualized through application of NBT/BCIP. Non-coated wells were treated as control.

### Analysis of biosilica formation in silicatein-coated culture wells

To synthesize biosilica within coated 6-well plates, 200 µM orthosilicate (prehydrolyzed TEOS) in 50 mM Tris-HCl (pH 7.4; 150 mM NaCl) are added (300 µl/well final volume) per well and incubated at 20°C for 0-4 h, typically for 4 h. To evaluate progression of the enzymatic reaction, aliquots of the solution are taken, after moderate agitation, to detect and quantify biosilica formation using the following techniques:
Analytical quantification: Aliquots (1 ml) are taken from the assays after the indicated period of time and centrifuged (10,000 x g, 30 min, 4°C). Pelleted silica particles are washed thrice with ethanol and, then, hydrolyzed with 2 M NaOH for 30 min (4°C). Subsequently, the concentration of silicic acid in solution is quantified colorimetrically by molybdenum blue procedure, using the "Silicon Test Colorimetric Assay Kit" and a silicon standard for calibration. Based on the absorbance at 650 nm, absolute amounts of silicic acid are calculated. As a negative control, non-coated culture wells are incubated with 200 µM orthosilicate and processed in the same way.
Fluorescence staining: to trace the formation of poly(silicate) microscopically, the fluorophore Rhodamine 123 is employed. Aliquots of the reaction mixture (100 µl [floating biosilica]) are removed from the wells and 20 µl of Rhodamine 123 (10 µg/ml dimethyl sulfoxide) are added. 10 min later, the samples are inspected with an AHBT3 light microscope, including an AH3-RFC reflected light fluorescence attachment (Olympus). Wavelengths of 490 nm (excitation) and 520 nm (emission) are used.

For energy dispersive X-ray spectroscopy (EDX) analysis, the bottoms of the culture plates are removed, washed extensively with distilled water to eliminate excess buffer salts, and placed onto grids to visualize the substratum-bound biosilica.

Scanning and transmission electron microscopy (SEM and TEM): During the formation of biosilica in silicatein-coated wells, part of the siliceous product firmly attached to the bottom of the plates and could only be detached with a scraper while the rest of the siliceous product was only loosely attached and could be removed by slight agitation after the indicated times. The samples thus obtained are carefully washed, air-dried, and analyzed with a high resolution field emission scanning electron microscope. Alternatively, a transmission electron microscope is employed.

### Mineralization by SaOS-2 cells in vitro

Recombinant silicatein is used to coat plastic cover slips (Nunc) that have been placed into 24-well plates (14.5 mm well diameter; Orange Scientifique). Then, the plates are incubated for 4 h with 500 µl of orthosilicate (0-200 µM), as described above. Afterwards, 2 ml of SaOS-2 cells (5 x 10⁵ cells/ml McCoy's medium, containing 5% FCS, 50 µM ascorbic acid, and 5 mM ß-glycerophosphate) are added and cultivated for up to 7 days. Subsequently, the cover slips are removed and treated either with 10% Alizarin Red S (detection of calcific deposition and matrix mineralization) or with 10% Alcian Blue (cartilage proteoglycans and sulfated glycosaminoglycans), or with a combination of both dyes.

In a further set of experiments, SaOS-2 cells (5 x 10⁴ cells) were seeded on Glu-tag-silicatein/biosilica coated Ca-P cover slips, uncoated Ca-P cover slips, or on plain glass cover slips. To induce mineralization, cells were incubated for up to 7 days in McCoy's medium (containing 15% FCS, 50 µM ascorbic acid and 5 mM ß-glycerophosphate). Subsequently, cells were fixed and stained with 10% Alizarin Red S as described. Alizarin Red S was applied to determine quantitatively the amount of HA. In short, the slips with SaOS-2 cells were immersed into acetic acid. Then, the cells were mechanically removed and pelleted. Afterwards, the supernatant was supplemented with ammonium hydroxide to neutralize the acid and the optical density was read at 405 nm. The moles of bound Alizarin Red S were determined after generating a calibration curve. Values were normalized to total DNA amount that had been measured in parallel cultures, using a PicoGreen approach. Calf thymus DNA was used as a standard.

### Quantitative real-time RT-PCR (qRT-PCR) analysis

SaOS-2 cells are transferred into 24-well plates, either on silicatein/biosilica coated or uncoated Ca-P cover slips or on glass cover slips, or are transferred into 6-well plates, coated either with silicatein alone (silicatein-coated) or with silicatein/biosilica. After incubation in McCoy's medium (containing 5% FCS, 50 µM ascorbic acid, and 5 mM ß-glycerophosphate) for 1, 3, or 7 days, the cells are harvested, and total RNA is extracted following state-of-the-art techniques. To remove possible DNA contamination, the samples (5 µg) are treated with 1 U of DNAse in RT buffer (20 µl). First strand cDNA is synthesized by using the M-MLV reverse transcriptase (Promega). Each reaction (40 µl) contains approximately 5 µg of total RNA, 0.5 mM dNTPs, 100 pmol of oligo(dT)₁₈, and 400 U reverse transcriptase in RT buffer. Reactions are incubated at 42°C for 1 hr, followed by inactivation of the reverse transcriptase (65°C, 15 min). Subsequently, quantitative real-time PCR (qPCR) experiments are performed in an iCycler (Bio-Rad), using 1/10 serial dilutions in triplicate following state-of-the-art techniques. Each of the reverse transcriptase reaction mixtures is diluted as required. Then, 2 µl of the appropriate dilution are employed as template during 30 µl qPCR assays. Each reaction contained "Absolute Blue SYBR Green" master mixture (ABgene) and 5 pmol of each primer. All reactions are run with an initial denaturation at 95°C for 3 min, followed by 40 cycles, each with 95°C for 20 sec, 58°C for 20 sec, 72°C for 20 sec, and 80°C for 20 sec. Fluorescence data are collected at the 80°C step. The following primers are used for amplification, for the house keeping gene GAPDH (glyceraldehyde 3-phosphate dehydrogenase, GenBank accession number NM_002046.3) Fwd: 5'-ACTTTGTGAAGCTCATTTCCTGGTA-3' [nt₁₀₁₉ to nt₁₀₄₃] and Rev: 5'-TTGCTGGGGCTGGTGGTCCA-3' (nt₁₁₁₇ to nt₁₁₃₆) (product size 118 bp); RANKL (AF019047) Fwd: 5'-AGAGCGCAGATGGATCCTAA-3' [nt₃₃₉ to nt₃₅₈] and Rev: 5'-TTCCTTTTGCACAGCTCCTT-3' [nt₄₉₉ to nt₅₁₈] (180 bp); OPG (U94332.1) Fwd: 5'-GCAGCGGCACATTGGAC-3' [nt₉₃₇ to nt₉₅₃] and Rev: 5'-CCCGGTAAGCTTTCCATCAA-3' [nt₉₈₆ to nt₁₀₀₅] (69 bp); for *BMP2* (NM_001200.2) Fwd: 5'-ACCCTTTGTACGTGGACTTC-3' [nt₁₆₈₁ to nt₁₇₀₀] and Rev: 5'-GTGGAGTTCAGATGATCAGC-3' [nt₁₇₈₅ to nt₁₈₀₄] (124 bp) and *TRAP*/*ACP5* (AAH25414) Fwd: 5'-GATCTCCAAGCGCTGGAAC-3' [nt₄₆₂ to nt₄₈₀], and Rev: 5'-CTGAGTTGCCACATAGTGTC-3' [nt₅₆₀ to nt₅₈₀] (119 bp). The threshold position is set to 50.0 RFU [relative fluorescence units] above PCR subtracted baseline for all runs. Mean Ct values and efficiencies are calculated by the iCycler software (Bio-Rad, Hercules, CA; USA). Expression levels of the respective transcripts are correlated to the reference gene *GAPDH* to determine relative expression [e.g. E*_{GAPDH}* ^{Ct *GAPDH*}/E*_{RANKL}* ^{Ct *RANK*}, or E*_{GAPDH}* ^{Ct *GAPDH*}/E*_{OPG}* ^{Ct *OPG*}] where "E" describes PCR efficiency and "Ct" represents the threshold cycle.

### OPG and RANKL ELISA assays

8 x 10⁵ SaOS-2 cells in McCoy's medium (supplemented with 5% FCS, 50 µM ascorbic acid, and 5 mM β-glycerophosphate) are seeded in 6-well plates that had been coated with silicatein/biosilica or have remained untreated, as described above. After 10 min up to 7 days, culture medium aliquots of 1 ml are taken and stored at -20°C for detection of OPG or RANKL. In the cultures the cell number are determined by using MTS ([3-(4,5-dimethylthiazol-2-yl)-5-(3-carboxymethoxyphenyl)-2-(4-sulfophenyl)-2H-tetrazolium] 290 µg/ml) (Promega) and PES (phenazine ethosulfate; 30 µM; Sigma). Following incubation for 60 min, the optical density is determined at 530 nm (650 nm as reference). Subsequently, the cell number is calculated by linear interpolation, after creating a standard curve.

OPG and RANKL protein concentrations in culture medium are determined by ELISA, employing goat anti-human OPG/TNFRSF11 B antibody (R&D Systems) or goat anti-human TRANCE/RANKL/TNFSF11 antibody (R&D Systems). For this purpose, polyclonal antibodies (200 ng/ml in 0.1 M sodium bicarbonate; pH 8.6) are adsorbed to a 96-well ELISA plate at 4°C overnight. For calibration and standard curves, recombinant human OPG or RANKL (R&D Systems) are used, diluted in 100 mM Tris-HCl buffer (pH 7.4; 8% [v/v] skimmed milk). Captured proteins are detected with biotinylated antibodies (R&D Systems), 2 µg/ml ExtrAvidin peroxidase, and the chromogenic substrate 3,3',5,5'-tetramethylbenzidine (TMB, Sigma). After termination of the reaction with sulfuric acid, optical densities are determined at 450 nm.

### SDS-PAGE and Western blotting analyses

Proteins are subjected to SDS-PAGE, transferred to PVDF membranes, and probed for with anti-silicatein- or anti-silintaphin-1 antibodies. The resulting immunocomplexes are visualized by successive incubation with species-specific HRP-coupled secondary antibodies, and the "BM Chemiluminescence Western Blotting Substrate" (Roche Applied Science). In control experiments, antibodies are adsorbed to recombinant protein prior to their use.

Polyclonal antibodies against purified silicatein and silintaphin-1 can be raised in rabbits and monoclonal antibodies against purified silicatein and silintaphin-1 can be raised in female Balbc/An mice according to state-of-the-art procedures.

## Claims

1. Injectable material or material to be used as a drug or food supplement for the prophylaxis or treatment of osteoporosis, consisting of an enzyme or protein involved in biosilica (amorphous, hydrated silicon oxide) metabolism, such as silicatein or silintaphin-1 or silicatein-silintaphin-1 fusion proteins or combinations thereof, wherein said protein is provided with an N-terminal or C-terminal bound apatite (calcium phosphate) or silica-binding oligocationic or oligoanionic tag, or a multiple thiol groups carrying tag.

2. Material according to claim 1, wherein the oligocationic or oligoanionic tag consists of an oligo(amino acid), which preferably is composed of glutamic acid, aspartic acid or lysine or mixtures of these amino acids, for example of a length of six to ten amino acids.

3. Material according to claim 1, wherein the multiple thiol groups carrying tag consists of an oligo(cysteine) residue.

4. Material according to claim 1, wherein the enzyme or protein involved in biosilica metabolism carries a tag on both its N-terminus and C-terminus, whereby the chemical nature of these two tags (oligocationic or oligoanionic tag, or multiple thiol groups carrying tag) is either different or the same.

5. Material according to any of claims 1 to 4, wherein a silicatein polypeptide or a silicatein fusion protein is used, which has been produced using a prokaryotic or eukaryotic expression system, or which has been produced synthetically.

6. Material according to any of claims 1 to 5, wherein a silintaphin-1 polypeptide or a silintaphin-1 fusion protein is used, wherein said silintaphin-1 polypeptide or a polypeptide being homologous thereto in the amino acid sequence of the silintaphin-1 domain exhibits a sequence similarity or identity of at least 25% to the sequence shown in SEQ ID Nr. 3, or parts thereof.

7. Material according to any of claims 1 to 5, wherein a silicatein polypeptide is used, or a polypeptide being homologous thereto, which in the amino acid sequence of the silicatein domain exhibits a sequence similarity or identity of at least 25% to the sequence shown in SEQ ID Nr. 1, or parts thereof.

8. Material according to any of claims 1 to 7, **characterized in that** the tagged silicatein molecules or tagged silintaphin-1 molecules are bound via their tag to hydroxyapatite (HA) nanoparticles or HA - collagen nanoparticles.

9. Material according to any of claims 1 to 8, which has been modified ex vivo by biosilica formation using its silicatein or silicatein fusion protein component and a suitable biosilica precursor, wherein the biosilica precursor preferably consists of water glass, orthosilicic acid, orthosilicates, monoalkoxysilanetriols, dialkoxysilanediols, dialkoxysilanols, trialkoxysilanols, tetraalkoxysilanes, alkyl- or aryl-silanetriols, alkyl- or aryl-silanediols, alkyl- or aryl-monoalkoxysilanediols, alkyl-or aryl-monoalkoxysilanols, alkyl- or aryl-dialkoxysilanols, alkyl- or aryl-trialkoxysilanes, or silicon-catecholate complexes.

10. Material according to any of claims 1 to 9, which has been modified ex vivo by binding of nanoparticulate silica particles (nanosilica) or microparticulate silica particles or other metal oxide particles or mixtures thereof to its silintaphin-1 or silintaphin-1 fusion protein component.

11. Material according to any of claims 1 to 10, which has been modified ex vivo by binding of iron [iron (II) and/or iron (III)] to the multiple thiol groups carrying tag.

12. Material according to any of claims 1 to 11, consisting of the polymeric inorganic components (biosilica, oligo- and polysilicates), separated from the organic component.

13. Material according to any of claims 1 to 12, whereby one or more of the enzyme or protein components (silicatein or silintaphin-1 or silicatein-silintaphin-1 fusion proteins or combinations thereof) and/or the biosilica precursor or oligo/polysilicate has/have been encapsulated in a organic polymer, wherein the organic polymer preferably is poly(lactic acid).

14. Material according to any of claims 1 to 13 for the treatment of patients suffering from bone disorders, in particular osteoporosis, preferably for percutaneous injection into the fractured vertebra of osteoporotic patients by means of one or two bone biopsy needles (vertebroplasty and kyphoplasty).

15. Material according to claim 14 for the formulation of an orally or parenterally-administrable drug for prophylaxis or therapy of bone disorders, in particular osteoporosis.

16. Material according to any of claims 1 to 15 as a food supplement for prophylaxis or therapy of bone disorders, in particular osteoporosis, preferably as a supplement to yoghurt or other milk products for prophylaxis or therapy of bone disorders, in particular osteoporosis, wherein said material preferably is silica (biosilica), in particular polysilicate ("biosilicate") or nanoparticulate biosilica ("nanobiosilica"), or poly(lactic acid) encapsulated silica (biosilica).

17. Material according to claim 16 in combination with further resorbable additives, in particular polyphosphate, as a supplement to yoghurt or other milk products for prophylaxis or therapy of bone disorders, in particular osteoporosis.
